(19) Europäisches Patentamt
European Patent Office
Office européen des brevets

(11) EP 4 043 436 B1

(12) EUROPEAN PATENT SPECIFICATION

(45) Date of publication and mention of the grant of the patent:
**20.05.2026 Bulletin 2026/21**

(21) Application number: **20861878.5**

(22) Date of filing: **27.07.2020**

(51) International Patent Classification (IPC):
***C07D 211/62*** (2006.01) ***C07D 401/04*** (2006.01)
***C07D 401/06*** (2006.01) ***C07D 413/04*** (2006.01)
***A61K 31/445*** (2006.01) ***A61K 31/454*** (2006.01)
***A61P 3/00*** (2006.01) *A61P 1/00* (2006.01)
*A61P 1/02* (2006.01) *A61P 1/08* (2006.01)
*A61P 1/14* (2006.01) *A61P 1/16* (2006.01)
*A61P 3/04* (2006.01) *A61P 3/10* (2006.01)
*A61P 9/00* (2006.01) *A61P 11/02* (2006.01)
*A61P 13/08* (2006.01) *A61P 13/10* (2006.01)
*A61P 13/12* (2006.01) *A61P 15/00* (2006.01)
*A61P 17/00* (2006.01) *A61P 17/02* (2006.01)
*A61P 17/10* (2006.01) *A61P 19/02* (2006.01)
*A61P 25/00* (2006.01) *A61P 25/04* (2006.01)

(52) Cooperative Patent Classification (CPC):
**C07D 211/62; A61P 1/00; A61P 1/02; A61P 1/08; A61P 1/14; A61P 1/16; A61P 3/00; A61P 3/04; A61P 3/10; A61P 9/00; A61P 11/02; A61P 13/08; A61P 13/10; A61P 13/12; A61P 15/00;** (Cont.)

(86) International application number:
**PCT/CN2020/104989**

(87) International publication number:
**WO 2021/042911 (11.03.2021 Gazette 2021/10)**

(54) **N-PHENYL-1-BENZOYL-4-PIPERIDINECARBOXAMIDE DERIVATIVES AS MONOACYLGLYCEROL LIPASE (MAGL) INHIBITORS FOR THE TREATMENT OF METABOLIC DISORDERS**

N-PHENYL-1-BENZOYL-4-PIPERIDINECARBOXAMID-DERIVATE ALS MONOACYLGLYCEROL LIPASE (MAGL) INHIBITOREN ZUR BEHANDLUNG VON STOFFWECHSELERKRANKUNGEN

DÉRIVÉS DE N-PHÉNYL-1-BENZOYL-4-PIPÉRIDINECARBOXAMIDE EN TANT QU'INHIBITEURS DE LA LIPASE DE MONOACYLGLYCEROL (MAGL) POUR LE TRAITEMENT DE MALADIES MÉTABOLIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.09.2019 CN 201910836454**
**10.09.2019 CN 201910856962**
**10.09.2019 CN 201910854235**

(43) Date of publication of application:
**17.08.2022 Bulletin 2022/33**

(73) Proprietor: **Lunan Pharmaceutical Group Corporation**
**Linyi, Shandong 276006 (CN)**

(72) Inventors:
- **ZHANG, Guimin**
  **Shandong 276006 (CN)**
- **WANG, Jinxin**
  **Nanjing, Jiangsu 211198 (CN)**
- **YAO, Jingchun**
  **Shandong 276006 (CN)**
- **ZHAO, Guifang**
  **Shandong 276006 (CN)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 043 436 B1

(74) Representative: **Reddie & Grose LLP**
**The White Chapel Building**
**10 Whitechapel High Street**
**London E1 8QS (GB)**


(56) References cited:

**WO-A1-2013/131609 WO-A1-2015/149607**
**WO-A1-2017/049295 WO-A1-2017/049295**
**WO-A1-2018/129381 WO-A1-2019/105915**
**WO-A1-2019/115660 WO-A1-2020/035424**
**WO-A2-2006/069805 WO-A2-2012/170098**
**WO-A2-2015/048570 CN-A- 101 128 427**
**CN-A- 105 906 552 CN-A- 105 916 841**
**CN-A- 109 311 868 CN-A- 109 593 089**
**CN-A- 109 593 089 US-A1- 2016 214 951**
**US-A1- 2017 313 681 US-A1- 2018 079 756**
**US-A1- 2019 202 817**

- **XIE Y ET AL: "Discovery of potent non-urea inhibitors of soluble epoxide hydrolase", BIOORGANIC & MEDICINAL CHEMISTRY LETTERS, ELSEVIER, AMSTERDAM NL, vol. 19, no. 8, 15 April 2009 (2009-04-15), pages 2354 - 2359, XP026079472, ISSN: 0960-894X, [retrieved on 20080920], DOI: 10.1016/J.BMCL.2008.09.066**
- **ZHI ZHUOER ET AL: "Discovery of Aryl Formyl Piperidine Derivatives as Potent, Reversible, and Selective Monoacylglycerol Lipase Inhibitors", JOURNAL OF MEDICINAL CHEMISTRY, vol. 63, no. 11, 20 May 2020 (2020-05-20), US, pages 5783 - 5796, XP055788363, ISSN: 0022-2623, DOI: 10.1021/acs.jmedchem.9b02137**
- **ZHI ZHUOER, ZHANG WENTING, YAO JINGCHUN, SHANG YANGUO, HAO QINGJING, LIU ZHONG, REN YUSHAN, LI JIE, ZHANG GUIMIN, WANG JINXIN: "Discovery of Aryl Formyl Piperidine Derivatives as Potent, Reversible, and Selective Monoacylglycerol Lipase Inhibitors", JOURNAL OF MEDICINAL CHEMISTRY, AMERICAN CHEMICAL SOCIETY, vol. 63, no. 11, 11 June 2020 (2020-06-11), pages 5783 - 5796, XP055788363, ISSN: 0022-2623, DOI: 10.1021/acs.jmedchem.9b02137**
- **DATABASE Registry 20 June 2019 (2019-06-20), ..: "-4-Piperidinecarboxamide, N-(3-chlorophenyl)-1-(1H-indol-2-ylcarbonyl)-", XP055788395, retrieved from STN Database accession no. 2340481-37-8**
- **DATABASE Registry 28 June 2019 (2019-06-28), ..: "-4-Piperidinecarboxamide, N-(3,5-dimethylphenyl)-1-(1H-indol-2-ylcarbonyl)-", XP055788407, retrieved from STN Database accession no. 2347870-36-2**
- **DATABASE REGISTRY 24 June 2019 (2019-06-24), .: "4-Piperidinecarboxamide, 1-(1H-indol-2-ylcarbonyl)-N-(3-methoxyphenyl)", XP55788418, retrieved from STN Database accession no. 2344310-62-7**
- **DATABASE REGISTRY 23 June 2019 (2019-06-23), .: "4-Piperidinecarboxamide, N-(3,4-dichlorophenyl)-1-(1H-indol-2-ylcarbonyl)", XP055788480, retrieved from STN Database accession no. 2343019-98-5**
- **DATABASE REGISTRY 18 June 2019 (2019-06-18), . .: "4-Piperidinecarboxamide, N-(3,5-dichlorophenyl)-1-(1H-indol-2-ylcarbonyl)", XP055788475, retrieved from STN Database accession no. 2338189-38-9**
- **DATABASE REGISTRY 22 April 2018 (2018-04-22), .: "4-Piperidinecarboxamide, 1-([1,1'-biphenyl]-4-ylcarbonyl)-N-(3-chlorophenyl)", XP055788467, retrieved from STN Database accession no. 2217201-40-4**
- **DATABASE REGISTRY 22 April 2018 (2018-04-22), .: "4-Piperidinecarboxamide, N-(3,4-dichlorophenyl)-1-(3-methoxybenzoyl)", XP055789045, retrieved from STN Database accession no. 2217030-90-3**
- **DATABASE REGISTRY 22 April 2018 (2018-04-22), .: "4-Piperidinecarboxamide, 1-([1,1'-biphenyl]-4-ylcarbonyl)-N-(3-methoxyphenyl)", XP055789054, retrieved from STN Database accession no. 2217274-23-0**
- **DATABASE REGISTRY 22 April 2018 (2018-04-22), ..: "4-Piperidinecarboxamide, 1-([1,1'-biphenyl]-4-ylcarbonyl)-N-(3,4-dichlorophenyl)", XP055789067, retrieved from STN Database accession no. 2217251-42-6**
- **DATABASE REGISTRY .: "4-Piperidinecarboxamide, 1-([1,1'-biphenyl]-4-ylcarbonyl)-N-(3,5- dichlorophenyl)", XP055789075, retrieved from STN**
- **DATABASE REGISTRY 17 April 2018 (2018-04-17), .: "4-Piperidinecarboxamide, 1-([1,1'-biphenyl]-4-ylcarbonyl)-N-(3,5-dimethylphenyl)", XP055788465, retrieved from STN Database accession no. 2214031-19-1**
- **DATABASE REGISTRY 21 February 2018 (2018-02-21), "4-Piperidinecarboxamide, 1-[(5-fluoro-1H-indol-2-yl)carbonyl]-N-phenyl", XP055788462, retrieved from STN Database accession no. 2177530-54-8**
- **DATABASE REGISTRY 12 September 2016 (2016-09-12), .: "4-Piperidinecarboxamide, 1-(3-methoxybenzoyl)-N-(2-methoxyphenyl)", XP055788443, retrieved from STN Database accession no. 1991951-96-2**
- **DATABASE REGISTRY 8 September 2016 (2016-09-08), .: "4-Piperidinecarboxamide, N-(4-chlorophenyl)-1-(3-methoxybenzoyl)", XP055788504, retrieved from STN Database accession no. 1989422-30-1**
- **DATABASE REGISTRY 29 August 2016 (2016-08-29), .: "4-Piperidinecarboxamide, N-(3-fluorophenyl)-1-(3-methoxybenzoyl)", XP055788513, retrieved from STN Database accession no. 1981837-69-7**




(Cont. next page)

- **DATABASE REGISTRY .: "4-Piperidinecarboxamide, 1-(4-chlorobenzoyl)-N-(3-methoxyphenyl)", XP055789081, retrieved from STN Database accession no. 905172-72-7**
- **DATABASE REGISTRY 22 September 2014 (2014-09-22), .: "4-Piperidinecarboxamide, 1-(2-naphthalenylcarbonyl)-N-phenyl", XP055788526, retrieved from STN Database accession no. 1624146-19-5**
- **DATABASE REGISTRY 22 September 2014 (2014-09-22), .: "4-Piperidinecarboxamide, 1-([1,1'-biphenyl]-4-ylcarbonyl)-N-phenyl", XP055789086, retrieved from STN Database accession no. 1624126-48-2**
- **DATABASE REGISTRY 31 August 2011 (2011-08-31), .: "4-Piperidinecarboxamide, 1-(1H-indol-2-ylcarbonyl)-N-phenyl", XP055788530, retrieved from STN Database accession no. 1325844-20-9**
- **DATABASE REGISTRY 18 May 2011 (2011-05-18), .: "4-Piperidinecarboxamide, 1-(2-hydroxybenzoyl)-N-(2-methoxyphenyl)", XP055788540, retrieved from STN Database accession no. 1296587-93-3**
- **DATABASE REGISTRY 29 August 2006 (2006-08-29), .: "4-Piperidinecarboxamide, 1-(4-chlorobenzoyl)-N-(3,4-dichlorophenyl)", XP055788542, retrieved from STN Database accession no. 905172-26-1**
- **DATABASE REGISTRY .: "4-Piperidinecarboxamide, 1-(4-chlorobenzoyl)-N-(3,5-dichlorophenyl)", XP055789098, retrieved from STN Database accession no. 905171-41-7**
- **DATABASE REGISTRY 10 October 2005 (2005-10-10), .: "4-Piperidinecarboxamide, 1-(4-chlorobenzoyl)-N-(3-fluorophenyl)", XP055788548, retrieved from STN Database accession no. 864842-41-1**
- **DATABASE REGISTRY .: "4-Piperidinecarboxamide, 1-(4-chlorobenzoyl)-N-(4-chlorophenyl)", XP055789111, retrieved from STN Database accession no. 864840-27-7**
- **DATABASE REGISTRY 1 May 2003 (2003-05-01), .: "4-Piperidinecarboxamide, N-(3,5-dichlorophenyl)-1-(3-methoxybenzoyl)", XP055788552, retrieved from STN Database accession no. 508188-51-0**
- **DATABASE REGISTRY ANONYMOUS: "4-Piperidinecarboxamide, N-(3-chlorophenyl)-1-(3-methoxybenzoyl)", XP055789116, retrieved from STN Database accession no. 508188-16-7**
- **DATABASE REGISTRY 26 June 2002 (2002-06-26), .: "4-Piperidinecarboxamide, 1-(4-chlorobenzoyl)-N-(2-methoxyphenyl)", XP055788564, retrieved from STN Database accession no. 433952-89-7**
- **DATABASE REGISTRY 9 February 2016 (2016-02-09), .: "4-Piperidinecarboxamide, 1-(3-methoxybenzoyl)-N-(3-methoxyphenyl)", XP055788575, retrieved from STN Database accession no. 1985280-62-3**
- **DATABASE REGISTRY 31 October 2010 (2010-10-31), .: "4-Piperidinecarboxamide, N-(3-hydroxyphenyl)", XP055788569, retrieved from STN Database accession no. 1249010-19-2**
- **DATABASE REGISTRY 7 June 2009 (2009-06-07), .: "4-Piperidinecarboxamide, N-(4-chloro-3-fluorophenyl)", XP055788580, retrieved from STN Database accession no. 1153189-42-4**
- **DATABASE REGISTRY 23 April 2008 (2008-04-23), .: "4-Piperidinecarboxamide, N-(3-fluorophenyl)", XP55788587, retrieved from STN Database accession no. 1016532-14-1**
- **DATABASE REGISTRY 23 April 2008 (2008-04-23), .: "4-Piperidinecarboxamide, N-(3-chlorophenyl)-", XP055788585, retrieved from STN Database accession no. 1016748-08-5**
- **DATABASE REGISTRY 23 April 2008 (2008-04-23), .: "4-Piperidinecarboxamide, N-(3,5-dichlorophenyl)", XP055788591, retrieved from STN Database accession no. 1016530-58-7**

(52) Cooperative Patent Classification (CPC): (Cont.)
**A61P 17/00; A61P 17/02; A61P 17/10; A61P 19/02; A61P 25/00; A61P 25/04; A61P 25/08; A61P 25/14; A61P 25/16; A61P 25/18; A61P 25/20; A61P 25/22; A61P 25/24; A61P 25/28; A61P 25/32; A61P 29/00; A61P 31/12; A61P 35/00; A61P 35/02; A61P 37/06; A61P 37/08; C07D 401/04; C07D 401/06; C07D 413/04**

## Description

### FIELD OF TECHNOLOGY

The present invention relates to the field of medicine and in particular to a MAGL inhibitor for use in treating a MAGL-mediated disease or condition, and its preparation method.

### BACKGROUND

Monoacylglycerol Lipase (MAGL) is a kind of serine hydrolases which accelerate fat to be decomposed into glycerin and fatty acids, and are highly expressed in human aggressive tumor cells and primary tumor cells. High-level MAGL can maintain high pathogenicity of tumor cells by improving the level of fatty acids. MAGL has become an important target spot for the research and development of antitumor drugs.
WO 2017049295A1 discloses substituted 1-phenyl-3-(piperidin-4-yl) urea analogs as useful inhibitors of the DCN1-UBC12 interaction inhibitors of the DCN1-mediated cullin-RING ligase activity.
US 2017/313681 A1 discloses isoquinoline compounds for treating various diseases and pathologies.
Y. Xie et al. discloses a class of potent non-urea soluble epoxide hydrolase inhibitors, identified through high throughput screening and chemical modification.
WO 2018/129381 A1 discloses compounds that can bind to tubulin and activate GEF-H1 and methods for using these compounds to treat cancer.
US 2019/202817 discloses compounds that can be used as SGK1 and JNK inhibitors with antioxidant effect, which can be formulated into suitable formulations for treating a degenerative disease of central nervous system, or a brain tumor.
WO 2019/115660 A1 and WO 2019/105915 A1 disclose new heterocyclic compounds useful for MAGL inhibitors for the treatment of various diseases in a mammal.
Zhi Zhuoer et al., Discovery of Aryl Formyl Piperidine Derivatives as Potent, Reversible, and Selective Monoacylglycerol Lipase Inhibitors, J. Med. Chem., 2020 describes compounds synthesized as reversible inhibitors of MAGL, to support MAGL as a potential therapeutic target for depression.
WO2020/035424 describes heterocyclic compounds as MAGL inhibitors for the treatment or prophylaxis of neuroinflammation, neurodegenerative diseases, pain, cancer, mental disorders, multiple sclerosis, Alzheimer's disease, Parkinson's disease, amyotrophic lateral sclerosis, traumatic brain injury, neurotoxicity, stroke, epilepsy, anxiety, migraine and/or depression in a mammal.
CN109593089 describes the design and synthesis of 1-oxo-2,8-diazaspiro[4.5]decane piperidine formic acid amide compounds and their antimicrobial activity.
WO2015/149607 describes a class of tripeptide epoxy ketones containing a heterocyclic ring in a peptide skeleton and use of these compounds for an antitumor drug.
CN105906552 describes a method for synthesizing piperidine carboxylic acid amide pharmaceutical intermediate piperidine-4-formyl-(2,6-dimethyl)aniline .
WO2013/131609 describes triazolopyrazine derivatives as inhibitors of GCN2, for the treatment of cancer.
WO2006/069805 describes pyrido(3,2-D)pyrimidine derivatives useful for medical treatment.
WO2015/048570 describes EBI2 modulator compounds for the treatment of diseases or conditions such as, autoimmune diseases, cancer and cardiovascular diseases.

### SUMMARY

The present invention provides a compound of Formula I or a pharmaceutically acceptable salt thereof:

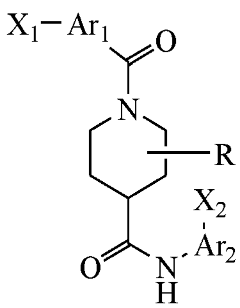

I ,

where, R is H or $C_{1-5}$ alkyl, and the $C_{1-5}$ alkyl is selected from methyl, ethyl, propyl, isopropyl, n-butyl, isobutyl, neo-butyl, n-pentyl, isopentyl, neo-pentyl, cyclopentyl, or $C_{1-5}$ alkyl substituted by halogen, hydroxy, carboxyl, $C_{1-3}$ alkyl, $C_{1-3}$ alkoxy, $C_{3-8}$ cycloalkyl, $C_{3-8}$ heterocyclyl, $C_{3-8}$ cycloalkoxy, aryl, and heteroaryl; the number of R is 0, 1, 2, 3 or 4;

In some embodiments, the $Ar_1$ is one or more of phenyl, naphthyl, phenoxy-phenyl and biphenyl;

the $X_1$ is hydroxy, at any substitution position;

the $Ar_2$ is benzene;

the $X_2$ is one or more of H, methyl, methoxy, hydroxy, F and Cl, at any substitution position.

In a specific embodiment, the $Ar_1$ has a substitution position of meta-position.

In some embodiments, the $Ar_1$ is one or more of benzofuryl, benzimidazolyl, benzothiophenyl, quinolyl, isoquinolyl and purinyl, at any substitution position;

the $X_1$ is one or more of H, methyl, methoxy, hydroxy, F, Cl and Br, at any substitution position;
the $Ar_2$ is phenyl;
the $X_2$ is one or more of H, methyl, methoxy, hydroxy, F and Cl, at any substitution position.

The present disclosure provides a compound of Formula b, and

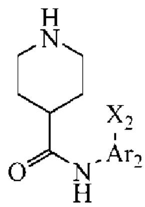

b ,

the compound is used for synthesizing the compound of Formula I, where, the $Ar_2$ and the $X_2$ are as described in Formula I.

In some embodiments of the disclosure, a fragment A within the dotted line in

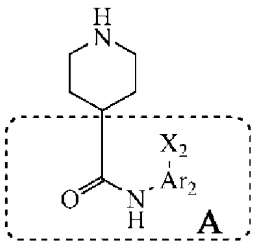

b

is substituted by

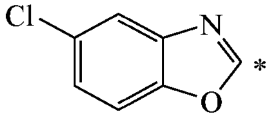

or

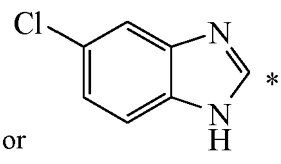

.

The present invention provides a method of synthesizing the compound of Formula I, including the following specific steps:

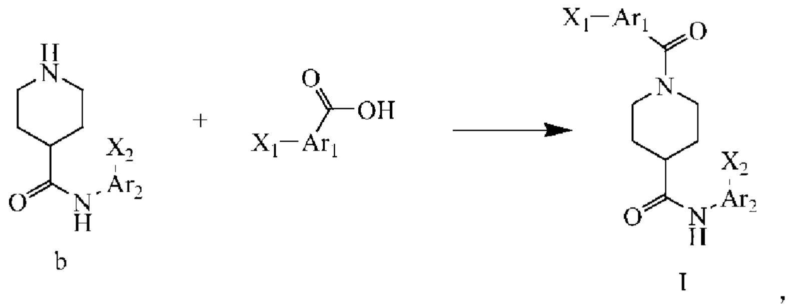

b or a salt of b is reacted with

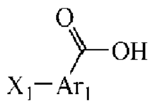

to obtain I; the $Ar_1$ and the $X_1$ are described in Formula I.

The b or the salt of b is reacted with

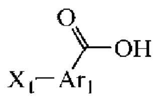

to obtain I; a condensating agent and an alkali are added in this step to facilitate the reaction; where, the condensating agent is selected from HBTU, DMC, HOBT, HOBT/EDCI, HATU, HATU/DIEPA, DCC, CDI, and isopropyl chloroformate; and the structural formula is respectively as follows:
the b or the salt of b is obtained by deprotection of a;

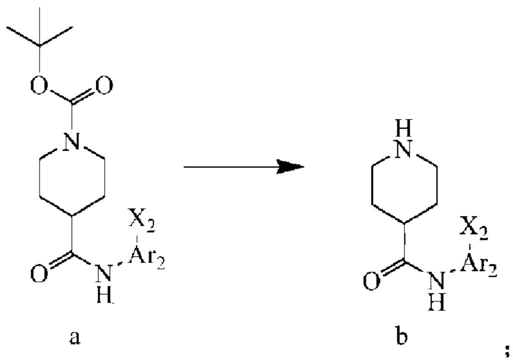

the alkali is an organic alkali, such as, N-diisopropylethylamine (DIPEA), diethylamine (DEA) or triethylamine (TEA).

The a or the salt of a is obtained by reacting sm with

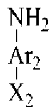

or a salt thereof;

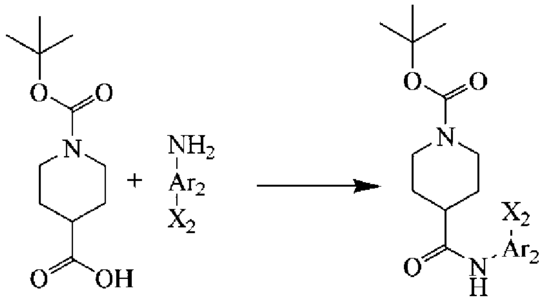

sm a

where, the sm may be prepared by the prior art, or synthesized by a person skilled in the art without any inventive efforts on the basis of the prior art and through limited tests. A condensating agent and an alkali are added in this step to facilitate the reaction; where, the condensating agent is selected from HBTU, DMC, HOBT, HOBT/EDCI, HATU, HATU/DIEPA, DCC, CDI, and isopropyl chloroformate.

In some specific embodiments, the following method may be taken:

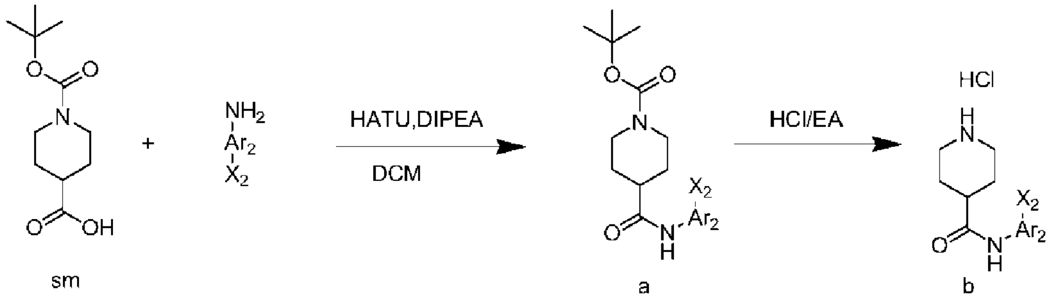


sm a b

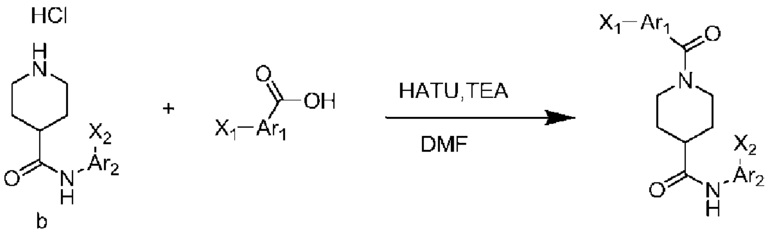


b

where, the reductant is preferably selected from at least one of $NaBH_4$, $KBH_4$, $NaBH_4/LiCl$.

Unless otherwise specified, solvents selected and used in the above or following steps herein are conventional solvents in the art, and the selection principle of the solvent is to dissolve reactants, but not participate in reaction, extract products or crystallize the corresponding products therein and separate the same from impurities, such as, water, halogenated hydrocarbons, alkyl amine, fatty hydrocarbons, esters, alcohols, aromatic hydrocarbons, ethers and heterocyclic solvents; specifically selected from, but not limited these solvents: methanol, ethanol, propanol, isopropanol, diethyl ether, ethyl acetate, acetic acid, cyclohexane, dichloromethane, trichloromethane, tetrahydrofuran, pyridine, diethylamine, triethylamine, dimethylformamide, methylbenzene and a mixture of at least two of them.

a, b and other serial number used herein are convenient for illustrating the general formula, and may be transformed into other number in detailed examples, such as, 1, 2, and 3, which are convenient for description, but free of influencing the structural formula and essence of the reaction equation thereof, and belong to the expression of the general formula and the reaction equation thereof. A person skilled in the art can judge that substituents of each intermediate in all the synthetic routes above are determined according to the structure of a target compound.

Compounds covered by the general formula of the target compound in general formula I and chiral isomers or cis-trans-isomers as well as a mixture of the isomers in any proportion in a specific substance representative thereof shall be within the scope of the compounds covered by the general formula of the target compound of general formula I and its specific substance representative thereof.

"At any substitution position" may be construed as any choice in case of a proper valence bond. For example, substitution on a benzene ring, may be one or more of ortho-position, meta-position, and para-position; for example, substitution in a heteroaromatic ring, may be one or more of 2-, 3-, 4-, 5- and other positions. In regard to the selection of

substituents, "one or more", or "a or a plurality of" refers to the number of substituents selected; and the specific number is one, two, three, four...specifically, for example, "$Ar_1$ is phenyl at any substitution position; $X_1$ is one or more of F and Cl at any substitution position" includes that the ortho (two), meta (two) and para (one) of phenyl may be substituted by one Cl, two Cl and three Cl or two meta-positions are respectively substituted by one Cl and one F or one Cl is substituted in the ortho position, while one F is substituted in the para position, and so on.

Unless otherwise specified, solvents selected and used in the above or following steps herein are conventional solvents in the art, and the selection principle of the solvent is to dissolve reactants, but not participate in reaction, extract products or crystallize the corresponding products therein and separate the same from impurities, such as, water, halogenated hydrocarbons, alkyl amine, fatty hydrocarbons, esters, alcohols, aromatic hydrocarbons, ethers and heterocyclic solvents; specifically selected from, but not limited these solvents: methanol, ethanol, propanol, isopropanol, diethyl ether, ethyl acetate, acetic acid, cyclohexane, dichloromethane, trichloromethane, tetrahydrofuran, pyridine, diethylamine, triethylamine, dimethylformamide, methylbenzene and a mixture of at least two of them.

Unless otherwise specified, in the above or following reaction herein, when there are excessive amount of reagents, substances capable of reacting with the excessive amount of reagents may be added at the end of the reaction for quenching the reaction. For example, water or saturated ammonium chloride may be used in some examples for quenching.

Unless otherwise specified, in the above or following reaction herein, the purification way of the product in each step of reaction is selected from extraction, crystallization, solvent removal and column chromatography; and the operations are conventional technologies in the art. Therefore, a person skilled in the art may deal with according to specific conditions.

The general formula of the present invention and synthesizing method of the general formula may derive these specific substances (not limited thereto). Moreover, under the guidance of the general formula of the present invention and synthesizing method of the general formula, specific compounds obtained by a person skilled in the art without any inventive efforts shall fall within the scope of the present invention.

Even though preferred embodiments of the present invention have been displayed and described here, it is obvious for a person skilled in the art that such kind of embodiment is merely provided by examples. Now, a person skilled in the art will envisage of lots of changes, variations and replacements not departing from the present invention. It should be understood that various replacements in embodiments of the present invention may be used for implementing the present invention. The claims annexed are aimed at defining the scope of the present invention such that methods, structures and other equivalent forms within the scope of these claims are covered.

The present invention provides a pharmaceutical composition, containing the above compound, namely, compounds covered by the general formula of the target compound of Formula I and a specific substance representative thereof, or a pharmaceutically acceptable salt thereof, and one or more pharmaceutically acceptable pharmaceutic adjuvants.

The present invention provides a pharmaceutical composition, containing the compound of the present application or a pharmaceutically acceptable salt and a pharmaceutically acceptable carrier thereof. The pharmaceutical composition includes but not limited to oral dosage forms, parenteral dosage forms, external formulations and rectal dosage forms. The composition may be a form of liquid, solid, semi-solid, gel or aerosol. In some embodiments, the pharmaceutical composition may be oral troches, capsules, pills, powders, sustained release preparations, solutions and suspensions, sterile solutions, suspensions or emulsions used for parenteral injection, ointments or creams for external use, or suppositories for rectal administration. In other embodiments, the pharmaceutical composition is a unit dosage form suitable for a single administration of an accurate dosage.

The present invention provides the compounds above or pharmaceutically acceptable salts thereof as well as a pharmaceutical composition thereof for use as a monoacylglycerol lipase inhibitor; or for use in treating a disease or a condition having pathological features of metabolic pathways of monoacylglycerol lipase.

The present invention provides the compounds above or pharmaceutically acceptable salts thereof or a pharmaceutical composition thereof for use in a method for inhibiting monoacylglycerol lipase. The method includes a method for inhibiting monoacylglycerol lipase in vitro and in vivo. The present invention further provides all the compounds above or pharmaceutically acceptable salts thereof as well as a pharmaceutical composition thereof for use in a method for treating a disease or a condition mediated by monoacylglycerol lipase. The present invention further provides a method for inhibiting MAGL in-vitro, comprising contacting the MAGL with the compounds above or pharmaceutically acceptable salts thereof.

The condition is selected from: metabolic disorders (e.g., obesity), nephrosis (e.g., acute inflammatory renal injury and diabetic nephropathy), emesis or vomiting (e.g., chemotherapy-induced emesis), nausea (e.g., refractory nausea or chemotherapy-induced nausea), eating disorders (e.g., anorexia or bulimia), neuropathy (e.g., diabetic neuropathy, pellagra neuropathy, alcoholic neuropathy, beriberi neuropathy), neurodegenerative conditions [multiple sclerosis (MS), Parkinson’s disease (PD), Huntington’s disease, dementia, Alzheimer’s disease, amyotrophic lateral sclerosis (ALS), epilepsy, frontotemporal dementia, sleep disorders, Creutzfeldt-Jakob Disease (CJD) or prion disease],

schizophrenia, depressive disorders, bipolar disorders, fremitus, dyskinesia, dystonia, spasticity, touretti's syndrome, abstinence syndromes [alcohol abstinence syndrome, antidepressant withdrawal syndrome, antipsychotic withdrawal syndrome, benzodiazepine withdrawal syndrome, Cannabis sativa abstinence syndrome, neonatal abstinence syndrome, nicotine abstinence syndrome, or opiates abstinence syndrome], traumatic brain injury, non-traumatic brain injury, spinal cord injury, epileptic seizure, conditions associated with abnormal cell growth or proliferation [e.g., benign tumors or cancers, such as, benign skin tumors, brain tumors, papilloma, prostate tumors, brain tumors (glioblastoma, medulloepithelioma, medulloblastoma, neuroblastoma, astrocytoma, astroblastoma, ependymoma, oligodendroglioma, choroid plexus tumor, neuroepithelioma, epiphyseal tumor, ependymoblastoma, malignant meningioma, sarcosis, melanoma and schwannoma), melanoma, metastatic tumor, renal cancer, bladder cancer, cerebral cancer, glioblastoma (GBM), gastrointestinal cancer, leukemia or blood cancer], inflammatory conditions [for example, appendicitis, bursitis, colitis, irritable bowel syndrome, ulcerative colitis, urocystitis, dermatitis, phlebitis, rhinitis, tendinitis, amygdalitis, vasculitis, acne vulgaris, chronic prostatitis, glomerulonephritis, hypersensitivity, IBS, pelvic inflammatory diseases, sarcoidosis, HIV encephalitis, rabies, brain abscess, neuroinflammation, central nervous system (CNS) inflammation], immune system conditions (e.g., transplant rejection or celiac disease), post-traumatic stress disorder (PTSD), acute stress disorder, panic disorder, substance-induced anxiety, obsessive-compulsive disorder (OCD), agoraphobia, specific phobia, social phobia, anxiety disorder, attention deficit disorder (ADD); attention deficit hyperactivity disorder (ADHD); pains [e.g., acute pain, chronic pain, inflammatory pain, visceralgia, post-surgical pain, migraine, low back pain, arthralgia, stomachache, stethalgia, post-mastectomy pain syndrome, menstrual pain, endometriosis pain, physical trauma-induced pain, headache, sinus headache, tension headache, arachnoiditis, herpes virus pain, diabetic pain, and pain induced by a condition selected from the following: osteoarthritis, rheumatoid arthritis, spondylitis, gout, labor pain, musculoskeletal diseases, dermatosis, toothache, heartburn, burn, sunburn, snake bite, venomous snake bite, spider bite, insect bites, neurogenic bladder, interstitial cystitis, urinary tract infection (UTI), rhinitis, contact dermatitis/hypersensitivity, itching, eczema, pharyngitis, mucositis, enteritis, irritable bowel syndrome (IBS), cholecystitis and pancreatitis; neuropathic pains (e.g., nervous lumbago, complex regional pain syndrome, pillar prosopalgia, causalgia, toxic neuropathy, reflex sympathetic dystrophy, diabetic neuropathy, chronic chemotherapeutant-induced neuropathy, or ischialgia)]; demyelinating diseases [e.g., multiple sclerosis (MS), Devic's disease, CNS neuropathy, central pontine myelinolysis, syphlitic myelopathy, leukoencephalopathy, leukodystrophy, Guillain-Barre syndrome, chronic inflammatory demyelinated polyneuropathy, anti-myelin-associated glycoprotein peripheral neuropathy, Charcot-Marie-Tooth Disease, peripheral neuropathy, myeleterosis, optic neuropathy, progressive inflammatory neuropathy, optic neuritis, and transverse myelitis] as well as cognitive impairments [e.g., Down's syndrome-associated cognitive impairment, Alzheimer's disease-associated cognitive impairment, PD-associated cognitive impairment, mild cognitive impairment (MCI), dementia, post-chemotherapy cognitive impairment (PCCI) and postoperative cognitive dysfunction (POCD)].

The present invention provides the compounds above or pharmaceutically acceptable salts thereof or a pharmaceutical composition thereof for use as a MAGL inhibitor; or for use in treating a disease associated with the MAGL inhibitor.

In some embodiments, the disease associated with the MAGL inhibitor is pain, migraine, irritable bowel syndrome or ulcerative colitis.

The pain in the above use is traumatic pain or pathologic pain. Further, the pathologic pain is inflammatory pain or endogenous pain. Furthermore, the pathologic pain is biogenic inflammatory pain, chemogenic inflammatory pain, blood-derived pain, immunogenic pain, endocrine-derived pain, metabolic lesion-caused pain, neuropathic pain or cardiogenic pain.

In the above use, the pain is transient pain, acute pain or chronic pain.

In the above use, the pain is nervous system pain, cardiovascular system pain, blood system pain, respiratory system pain, digestive system pain, endocrine system pain, urinary system pain, motor system pain or immune system pain.

In the above use, the migraine is common migraine, typical migraine and cluster migraine.

In the above use, the irritable bowel syndrome is constipation-type irritable bowel syndrome, diarrhea-type irritable bowel syndrome, and alternating constipation-diarrhea irritable bowel syndrome.

The MAGL inhibitor of the present invention can reduce the mice writhing times caused by acetic acid, extend latent time and delay a thermal stimulus pain threshold of mice pain induced by hot plate. The present invention has a significant analgesic effect.

In some embodiments, the MAGL inhibitor of the present invention can significantly delay the blood coagulation time of a migraine model, increase the times of tail suspension activity, and significantly increase the 5-HT level in brain tissues. The present invention shows a significant therapeutic effect on migraine.

The MAGL inhibitor of the present invention can significantly decrease the AWR scoring of an irritable bowel syndrome, reduce the 5-HT level of colon and blood serum. The above result hints that the present invention has a significant therapeutic effect on irritable bowel syndrome. Therefore, it is proved that such kind of compound has the bioactivity of preventing and treating irritable bowel syndrome.

The MAGL inhibitor of the present invention can significantly increase the colon length in an ulcerative colitis model and reduce total number of hematochezia, and decrease the IL-1 content.

## Some chemical terms

Unless otherwise specified, all the scientific and technological terms herein have the meanings the same as those understood by a person skilled in the art of the subject of the claims.

. Unless otherwise specified, a plural form is also included in case of using the singular in this present application. It should be further noted that unless otherwise specified, the "or", "either" denotes "and/or". Furthermore, the term, "comprise" and its other forms used herein, for example, "include", "contain" and "have" are non-restrictive descriptions.

Definitions on standard chemical terms may be found in references (including Carey and Sundberg "ADVANCED ORGANIC CHEMISTRY 4TH ED." Vols. A(2000) and B (2001), Plenum Press, New York). Unless otherwise specified, conventional methods within the technical scope of the art, for example, mass spectrometry, NMR, IR and UV/Vis spectroscopy and a pharmacology method are used. Unless otherwise specified, terms used in the related description of analytical chemistry, organic synthetic chemistry, medicine and pharmaceutical chemistry are known in the art. Standard technologies may be used in chemical synthesis, chemical analysis, medicament preparation, formulation and delivery as well as treatment on a patient. For example, reaction and purification may be implemented by means of a manufacturer's instruction for a kit, or according to a way commonly known in the art or by the description of the present application. Generally, the above technology and method may be implemented according to the description of multiple summary and more detailed literatures cited and discussed in the description by a conventional method known very well in the art. In this description, a person skilled in the art may choose a group and a substituent to provide a stable structural portion and a compound.

The term "alkyl" used herein refers to saturated linear or branched hydrocarbon. Exemplary alkyl includes, but not limited to, linear or branched hydrocarbons having 1-6, 1-4 or 1-3 carbon atoms, and respectively called $C_{1-6}$ alkyl, $C_{1-4}$ alkyl and $C_{1-3}$ alkyl herein. Exemplary alkyl includes, but not limited to, methyl, ethyl, propyl, isopropyl, 2-methyl-1-butyl, 3-methyl-2-butyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, tert-butyl, pentyl, isopentyl, neo-pentyl, hexyl, and the like.

The $C_{1-4}$ alkyl herein includes $C_{1-3}$ alkyl, $C_{1-2}$ alkyl, $C_{2-3}$ alkyl and $C_{2-4}$ alkyl; specific examples include but not limited to, methyl, ethyl, propyl, isopropyl, n-butyl, isobutyl and neo-butyl.

Alkoxy is, namely, alkyloxy. The $C_{1-3}$ alkoxy herein includes $C_{1-2}$ alkoxy and $C_{2-3}$ alkoxy; and specific examples include but not limited to, methoxy, ethoxy, propoxy and isopropoxy.

The term "cycloalkyl" used herein refers to saturated or partially unsaturated hydrocarbons, e.g., a monocyclic 5-8 membered ring system or a bicyclic 8-12 membered ring system. The $C_{3-8}$ cycloalkyl herein includes 3-8, 3-6, 4-6, 6-8, 3-7 or 5-8 carbon atoms, which is called $C_{3-8}$ cycloalkyl, $C_{3-6}$ cycloalkyl, or $C_{4-6}$ cycloalkyl herein, and only has different forms but same meanings. Exemplary cycloalkyl includes, but not limited to cyclohexyl, cyclopentyl, cyclopentenyl, cyclobutyl or cyclopropyl.

For example, the term "heterocyclyl" used herein contains a monocyclic 5-8 membered ring system or a bicyclic 8-12 membered ring system having one or more hetero atoms (such as, one to three hetero atoms, e.g., N, O or S).For example, the $C_{3-8}$ heterocyclyl herein represents a monocyclic or polycyclic group having 3 to 8 atoms, which contains $C_{3-6}$ heterocyclyl, $C_{4-8}$ heterocyclyl, $C_{5-8}$ heterocyclyl, $C_{4-7}$ heterocyclyl, $C_{3-7}$ heterocyclyl, $C_{6-8}$ heterocyclyl, and has at least one ring containing 1, 2, 3, or 4 heterocyclic atoms selected from N, O or S; the ring is not aromatic. Examples of heterocyclyl include but not limited to morpholinyl, sulfomorpholinyl, furyl, piperazinyl, piperidyl, pyranyl, pyrrolidyl, tetrahydropyranyl, tetrahydrofurfuryl and 1,3-dioxane.

$C_{3-8}$ cycloalkoxy, is namely cycloalkyloxy, and the cycloalkyl is paraphrased the same as that of the above cycloalkyl.

The term "aryl" used herein refers to an aromatic mono- or poly-carboatomic ring group having 6 to 12 carbon atoms of at least one aromatic ring. The $C_{6-10}$ aryl herein contains $C_{6-8}$ aryl, $C_{6-10}$ aryl, $C_{6-9}$ aryl, $C_{7-10}$ aryl, $C_{8-10}$ aryl, $C_6$ aryl, $C_7$ aryl, $C_8$ aryl, $C_9$ aryl, and $C_{10}$ aryl. A specific example of an unsubstituted "aryl"includes phenyl. Specific examples of a substituted "aryl" includes phenyl substituted by mono- or poly- hydrocarbyl, pentalenyl, indenyl, naphthyl, and azulenyl. Examples of substituted "aryl" also include tolyl, ethyl phenyl, dimethyl phenyl, propyl phenyl, isopropyl phenyl, methyl ethyl phenyl, trimethyl phenyl, butyl phenyl, methyl propyl phenyl, methyl isopropyl phenyl, diethyl phenyl, dimethyl ethyl phenyl, tetramethyl phenyl, tert-butyl phenyl, 1,2,3,4-tetrahydronaphthyl, 1,2-dihydronaphthyl, indanyl and 1H-indenyl.

The $C_{5-18}$ aryl herein includes $C_{6-10}$ aryl, $C_{11-12}$ aryl, $C_{13-15}$ aryl and $C_{16-18}$ aryl, such as, pentyl phenyl, methyl butyl phenyl, methyl isobutyl phenyl, methyl tert-butyl phenyl, ethyl propyl phenyl, ethyl isopropyl phenyl, methyl propyl phenyl, methyl isopropyl phenyl, trimethyl ethyl phenyl, pentamethyl phenyl, and the like.

The heteroaryl or heteroaryl ring or "heteromatic group" described herein refers to a monocyclic aromatic 5-8

membered ring system or a bicyclic 8-12 membered ring system having one or more hetero atoms (such as, one to three hetero atoms, e.g., N, O or S). 5 to 10-membered heteroaryl described herein contains 5 to 9-membered heteroaryl, 5 to 8-membered heteroaryl, 5 to 7-membered heteroaryl, 5 to 6-membered heteroaryl, 6 to 10-membered heteroaryl, 6 to 9-membered heteroaryl, 6 to 8-membered heteroaryl, 7 to 10-membered heteroaryl, 7 to 9-membered heteroaryl, 8 to 10-membered heteroaryl, 9 to 10-membered heteroaryl, 5-membered heteroaryl, 6-membered heteroaryl, 7-membered heteroaryl, 8-membered heteroaryl, 9-membered heteroaryl, and 10-membered heteroaryl. If possible, the heteroaryl ring may be linked to the adjacent group via C or N. Examples include but are not limited to: furyl, thienyl, 1H-1,2,3-triazolyl, 2H-1,2,3-triazolyl, pyrimidyl, pyrazinyl, 1,3,5-triazinyl, 1,2,4-triazinyl, 1,2,3-triazinyl, 1,2,4-oxadiazolyl, 1,3,4-oxadiazolyl; examples of 8-12 membered bicycloheteroaryl include but are not limited to: benzofuryl, isobenzofuryl, benzo[b]thienyl, benzo[c]thienyl, isoindolyl, benzo[d]isoxazolyl, benzo[c]isoxazolyl, benzo[d]oxazolyl, benzo[d]isothiazolyl, benzo[c]isothiazolyl, benzo[d]thiazolyl, benzo[d]imidazolyl, benzo[d]imidazolyl, and benzo[d][1,2,3]triazolyl, oxazolyl, thiazolyl, imidazolyl, pyrrolyl, pyrazolyl, isoxazolyl, isothiazolyl, 1,2,4-oxadiazole-5-yl, 1,2,4-oxadiazole-3-yl, 1,3,4-oxadiazolyl, 1,2,5-oxadiazolyl, 1,2,3-oxadiazolyl, 1,2,4-thiadiazole-5-yl, 1,2,4-thiadiazole-3-yl, 1,2,5-thiadiazolyl, 1,3,4-thiadiazolyl, 1,2,3-thiadiazolyl, 1,2,4-triazolyl, 1,2,3-triazolyl, tetrazolyl, indolyl, benzothiazolyl, benzoxazolyl, benzimidazolyl, benzisothiazolyl, imidazo[1,2-α]pyridyl, pyridazinyl, 1,2,4-oxadiazolyl, 1,2,4-thiadiazolyl, pyridyl and benzoisoxazolyl.

"One or more substituents of benzene, naphthalene..." or "one or more of benzene, naphthalene..." described herein refer to one or two or three of phenyl, naphthyl...

The term "acyloxy", namely, "acyl oxy" refers to a group -O-C(=O)-R; R is selected from H and substituted or unsubstituted $C_{1-5}$ alkyl, and the unsubstituted $C_{1-5}$ alkyl is selected from methyl, ethyl, propyl, isopropyl, n-butyl, isobutyl, neo-butyl, n-pentyl, iso-pentyl, and neo-pentyl; the substituted $C_{1-5}$ alkyl is $C_{1-5}$ alkyl substituted by halo, hydroxy, amino, carboxyl, $C_{1-3}$ alkyl, $C_{1-3}$ alkoxy, $C_{3-8}$ cycloalkyl, $C_{3-8}$ cycloalkyl, $C_{3-8}$ cycloalkoxy, aryl, and heteroaryl. In some embodiments, the R is n-propyl or

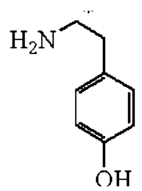

Halogen, the term used herein, "halo" or "halogen" refers to F, Cl, Br or I.

When a substituent is described by a conventional chemical formula written from left to right, the substituent also includes chemically equivalent substituents obtained by writing from right to left. For example, $CH_2O$ is equivalent to $OCH_2$.

The "compound" described herein refers to including all the stereoisomers, geometrical isomers, tautomers and isotopes. The compound of the present application may be asymmetrical, for example, having one or more stereoisomers. Unless otherwise specified, all the stereoisomers include, for example, enantiomers and diastereoisomers. The compound having asymmetrically substituted carbon atoms in the present application may be separated in an optically active pure form or in a racemic form. The optically active pure form may be split from a racemic mixture, or synthesized by chiral raw materials or chiral reagents. The compound of the present application further includes a form of tautomer. The form of tautomer is derived from an exchange of a single bond and adjacent double bonds accompanied with a proton migration. The compound of the present application further includes atoms of all the isotopes whatever it is an intermediate or a final compound. Atoms of isotopes include the same atomic number, but have different mass number. For example, isotopes of H include deuterium and tritium. That is, the compound of the present application includes compounds whose partial or complete hydrogen (H) atoms are replaced by tritium (T) and/or deuterium (D), and also includes compounds whose partial or complete $^{12}C$ are replaced by $^{13}C$ and/or $^{14}C$; as well as compounds replaced between other isotopes (e.g., N, O, P, and S), for example, $^{14}N$ and $^{15}N$; $^{18}O$ and $^{17}O$; $^{31}P$ and $^{32}P$; $^{35}S$ and $^{36}S$, and the like. The compound described herein may have one or more stereoisomeric centers; and each stereoisomeric center may exist in a R or an S configuration or a combination thereof. Similarly, the compound described herein may have one or more double bonds; and each double bond may exist in an E (trans-) or Z (cis-) configuration or a combination thereof. A specific stereoisomer, regioisomer, diastereoisomer, enantiomer, or an epimer should be construed as including all the possible isomers, such as, stereoisomer, constitutional isomer, diastereoisomer, enantiomer or epimer and a mixture thereof. Therefore, the compound described herein includes all the configurationally different stereoisomers, constitutional isomers, diastereoisomers, enantiomers or epimers and the corresponding mixtures thereof. A person skilled in the art knows the technology used for transforming a specific stereoisomer or keeping a specific stereoisomer the same, and the technology for splitting a stereoisomer mixture, and may choose a proper method based on a specific situation. See, for example, Fumiss et al.(eds.),VOGEL'S ENCYCLOPEDIA OF PRACTICAL ORGANIC CHEMISTRY 5.sup.TH ED., Longman Scientific and Technical Ltd., Essex, 1991, 809-816; and Heller, Acc. Chem. Res. 1990, 23, 128.

The term "optional/arbitrary" or "optionally/arbitrarily" refers that the subsequently described event or situation may occur or may not occur, and the description includes occurrence of the event or situation and not occurrence of the event or situation.

## Some pharmaceutical terms

The term "treating" used herein and other similar synonyms include relieving, alleviating or improving a disease or a condition, inhibiting a disease or a condition, for example, blocking the development of a disease or a condition, relieving a disease or a condition, making a disease or a condition improved, relieving a symptom caused by a disease or a condition, or suspending a symptom of a disease or a condition, preventing other symptoms, improving or preventing a potential metabolism reason for a symptom; further, the term includes a purpose of prevention. The term further includes obtaining a treatment effect and/or prevention effect. The treatment effect refers to healing or improving a potential disease treated. Furthermore, the healing or improvement of one or more physiological symptoms related to a potential disease is also a treatment effect, for example, even though a patient may be still influenced by a potential disease, the improved condition of the patient is observed. In term of a prevention effect, a patient having the risk of suffering from a specific disease is administered the composition, or even if no disease diagnosis is made, the composition is administered to the patient appearing one or more physiological symptoms of the disease.

The term "effective amount", "therapeutically effective amount" or "pharmaceutically effective amount" used herein refers to an amount of at least one active substance (e.g., the compound of the present application) which is enough to relieve one or more physiological symptoms of the disease or condition treated to some extent after taking. The result may be reduction and/or relieving of a sign, a symptom or a cause of a disease, or any other change required in biosystem. For example, the "effective amount" for treatment refers to an amount of the composition including the compound disclosed herein required by providing a significant relieving effect of a disease/condition clinically. A technology, for example, a dose escalation test, may be used to determine an effective amount suitable for any individual case.

The term, "taking", "applying", "administering", or the like used herein refers to methods capable of delivering a compound or a composition to a site demanded for biological action. These methods include but not limited to oral, duodenum, parenteral injection (including intravenous, subcutaneous, intraperitoneal, intramuscular, intra-arterial injection or infusion), external or rectal administration. A person skilled in the art knows application technologies capable of being used for the compound and method described herein, for example, those discussed in Goodman and Gilman, The Pharmacological Basis of Therapeutics, current ed.; Pergamon; and Remington's, Pharmaceutical Sciences (current edition), Mack Publishing Co., Easton, Pa.

The term "acceptable" used specific to the formulation, composition or component herein refers that the general health condition of a subject receiving treatment will be not harmfully influenced for a long time.

The term "pharmaceutically acceptable" used herein refers to a substance (e.g., a carrier or a diluent) not affecting bioactivity or properties of the compound of the present application, and non-toxic relatively; namely, the substance may be applied to an individual and free of causing undesirable biological reaction or free of interacting with any component contained in the composition via an undesirable way.

The term "pharmaceutical composition" used herein refers to a mixture of mixing the compound of the present application with at least one pharmaceutically acceptable substance. The pharmaceutically acceptable substance includes but not limited to a carrier, a stabilizer, a diluent, a dispersant, a suspending agent, a thickener and/or an excipient.

The term "carrier" used herein refers to a relatively non-toxic substance which helps to introduce the compound of the present application into cells or tissues.

The term "pharmaceutically acceptable salt" used herein refers to a salt which retains the biopotency of free acids and free alkali of a designated compound, and is free of adverse effect in biology or other aspects. The compound of the present application further includes a pharmaceutically acceptable salt. The pharmaceutically acceptable salt refers to a form where a base group in a parent compound is transformed into a salt. The pharmaceutically acceptable salt includes but not limited to base groups, such as, salts of inorganic or organic acids of amido (amino). The pharmaceutically acceptable salt of the present application may be synthesized by a parent compound, namely, a basic group in a parent compound is reacted with 1-4 equivalent amount of acids in a solvent system. A proper salt is enumerated in Remingtong's Pharmaceutical Sciences, 17th ed., Mack Publishing Company, Easton, Pa., 1985, p.1418 and Journal of Pharmaceutical Science, 66, 2(1977).

Unless otherwise specified, the salt in the present application refers to an acidic salt formed by an organic/inorganic acid, and a basic salt formed by an organic/inorganic base. Additionally, a zwitter-ion (inner salt) will be formed when the basic functional group of the compound in the general formula is pyridine or imidazole and the acidic functional group is carboxylic acid; the inner salt is also included in the salt of the present application.

## DETAILED DESCRIPTION OF THE EMBODIMENTS

The specific compound representative of the present invention may be synthesized by the synthesizing method of the general formula disclosed herein. The present invention will be further described by reference to detailed embodiments. Furthermore, under the guidance of the general formula, synthesizing method of the general formula and detailed embodiments of the present invention, a person skilled in the art may obtain other specific compounds without any inventive effort, and these compounds fall within the scope of the present invention.

### Example 1 Synthesis of

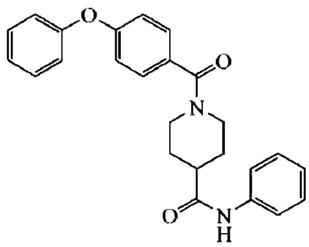

MAGLZ-II-01

Preparation of an intermediate a:

N-Boc-4 piperidine formic acid (458.6 mg, 2 mmol, 1eq) was dissolved into 15 ml DCM; DIPEA (0.7 ml, 4 mmol, 2eq) and HATU (912 mg, 2.4 mmol, 1.2eq) were added, and stirred for 10 min at room temperature. Phenylamine (0.365 ml, 4 mmol, 2eq) was added and stirred for 12 min at room temperature, then washed with water twice, and washed twice by 0.1 M diluted hydrochloric acid, then distilled under reduced pressure to remove the solvent to obtain an intermediate a without purification.

Preparation of an intermediate b:

The intermediate a was dissolved into 10 ml ethyl acetate, and 2 ml HCl/EA solution was added and stirred for 24 h at room temperature to separate out a white solid, then the white solid was subjected to suction filtration and washed by ethyl acetate to obtain an intermediate b, namely, white solid 347.5 mg (72.4%).

Preparation of MAGLZ-II-01:

The intermediate b (120 mg, 0.5 mmol, 1.5eq) was dissolved into 10 ml DMF, and TEA (0.23 ml, 1.65 mmol, 5eq) was added and stirred for 30 min at room temperature, then HATU (152 mg, 0.4 mmol, 1.2eq) and 4-phenoxybenzoic acid (71.4 mg, 0.33 mmol, 1eq) were added and stirred for 2 h at room temperature for full reaction; a saturated sodium chloride solution was added to the reaction solution, and ethyl acetate was used for extraction for 3 times, and organic phases were combined, and washed with a saturated sodium chloride solution for 3 times, and dried by anhydrous sodium sulfate, separated and purified by column chromatography (petroleum ether/ethyl acetate=1:1) to obtain a target compound, a light yellow solid 102.4 mg (76.8%).

$^1$HNMR (CDCl$_3$) δ(ppm):7.80-7.88(m, 1H),7.47-7.54(m,2H),7.29-7.39(m,5H),6.96-7.19(m,6H),2.79-3.05(m,2H ),2.45-2.58(m, 1H),1.65-2.06(m,6H). The MS result is equivalent to the theoretical value.

Preparation processes of Examples 2-23 were based on the synthesizing method of the general formula and similar to those of Example 1, where, b in Examples 22 and 23 was changed into

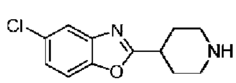 和 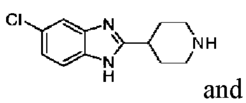 and

The MS result of each target product is equivalent to the theoretical value; and the NMR result is as follows:

| No. | Target product No. | Characterization data |
|---|---|---|
| **Example 2** | 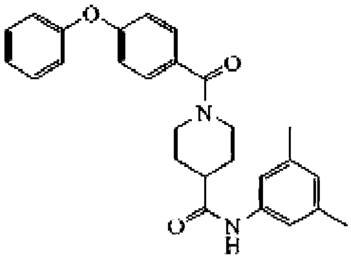 MAGLZ-II-02 | $^1$H NMR ($CDCl_3$) δ (ppm):7.32-7.42(m,4H),7.11-7.20(m,3H),6.95-7.0 7(m,4H),6.74(s,1H),2.79-3.05(m,2H),2.43-2,56(m ,1H),2.27(-s,6H),1.71-2.07(m,6H). |
| **Example 3** | 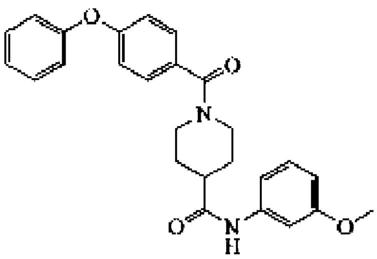 MAGLZ-II-03 | $^1$H NMR(300 MHz, DMSO-$d_6$) δ (ppm): 9.92 (s,1H), 7.40-7.47(m,4H), 7.32-7.34 (m,1H), 7.07-7.23 (m,5H), 7.00-7.05 (m,2H), 3.78-4.68 (m,2H), 3.72 (s,3H), 2.78-3.17 (m,2H), 2.57-2.68 (m,1H), 1.74-1.93 (m,2H), 1.53-1.68 (m,2H); |
| **Example 4** | 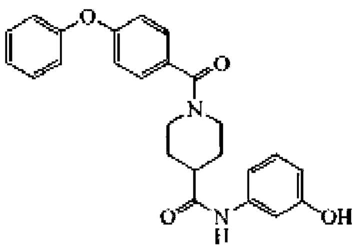 MAGLZ-II-04 | $^1$H NMR(DMSO$d_6$) δ (ppm): 9.83(s,1H),9.37(s,1H),7.40-7.46(m,4H),7.17-7.25( m,2-H),7.08-7.13(m,2H),7.00-7.06(m,3H),6.93-6.9 9(m,1H),6.38-6.45(m, 1H),2.88-2.92(m, 1H),2.68-2 .75(m,3H),2.56-2.66(m,1H), 1.75-1.93(m,2H), 1.50-1.65(m,2H) |
| **Example 5** | 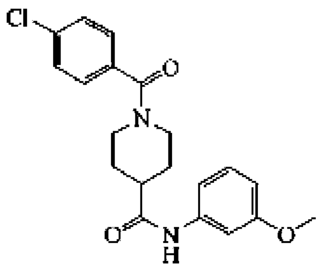 MAGLZ-II-05 | $^1$H NMR($CDCl_3$) δ (ppm):7.32-7.44(m,5H),7.17-7.25(m,1H),6.92-6.9 9(m,1H),6.65-6.71(m,1H),3.80(s,3H),2.73-3.31(m ,4-H),2.46-2.56(m,1H),1.78-1.97(m,4 H). |
| **Example 6** | 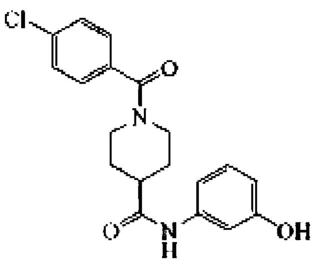 MAGLZ-II-06 | $^1$H NMR(DMSO$d_6$) δ (ppm):9.82(s,1H),9.38(s,1H),7.42-7.55(m,4H),7.1 6-7.20(m,1H),7.01-7.09(m,1H),6.93-6.99( m,1H),6.39-6.45(-m,1H),2.68-2.91(m,4H),2.55 -2.64(m,1H),1.80-2.02(m,2H),1.53-1.65(m,2H). |

(continued)

| No. | Target product No. | Characterization data |
|---|---|---|
| **Example 7** | 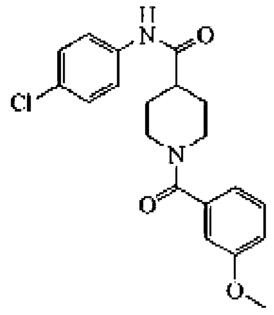 MAGLZ-II-07 | $^1H$ NMR($CDCl_3$) δ (ppm):7.70(s,1H),7.42-7.52(m,2H),7.21-7.37(m,3 H),6.92-6.98(m,2H),3.81(s,3H),2.80-2.99(m,4H), 2.45-2.55(m,1H),1.99-2.12(m,2H), 1.78-1.89(m,2H). |
| **Example 8** | 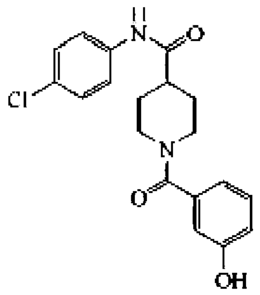 MAGLZ-II-08 | $^1H$ NMR(DMSO$d_6$) δ (ppm):10.08(s,1H),9.68(s,1H),7.60-7.66(m,2H),7. 32-7.38(m,2H),7.20-7.28(m,1H),6.73-6.86(m,3H), 2.67-2.93(m,3H),2.57-2.66(m, 1H), 1,68-2.00(m,3 H), 1.52-1.65(m,2H). |
| **Example 9** | 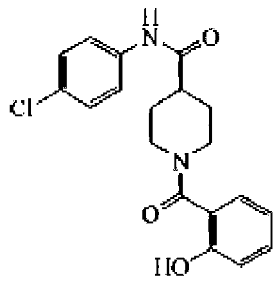 MAGLZ-II-09 | $^1H$ NMR(DMSO$d_6$) δ (ppm):10.02(s,1H),9.72(s,1H),7.60-7.66(m,2H),7. 31-7.37(m,2H),7.18-7.25(m,1H),7.08-7.13(m,1H), 6.81-6.89(m,2H),3.98-4.13(m,2H),2.85-2.97(m,2 H),2.54-2.66(m,1H),1.75-1.86( m,2H), 1.52-1.66(m,2H). |
| **Example 10** | 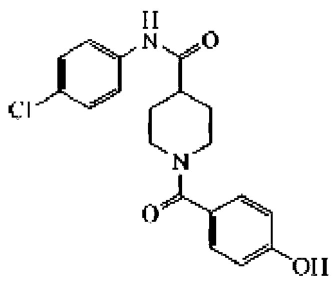 MAGLZ-II-10 | $^1H$ NMR(DMSO$d_6$) δ (ppm): 10.10(s,1H),9.87(s,1H),7.61-7.66(m,2H),7.33-7.3 8(m,2H),7.23-7.29(m,2H),6.77-6.83(m,2H),2.86-3 .02(m,2-H),2.54-2.85(m,3H),1.77-1.87(m,2H),1.52 -1.62(m,2H). |

(continued)

| No. | Target product No. | Characterization data |
|---|---|---|
| **Example 11** | 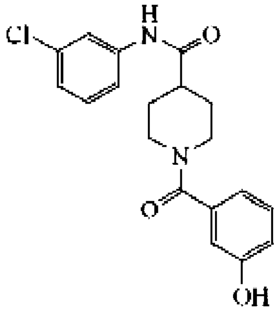 MAGLZ-II-11 | $^1$H NMR(DMSOd$_6$) δ (ppm): 10.08(s,1H), 9.62(s,1H),7.82(t,1H,J=3Hz),7.44-7.50 (m,1H),7.32(t,1H, J=6Hz), 7.24 (t,1H,J=6Hz), 7.07-7.12(m,1H), 6.74-6.86(m,3H), 2.89-2.98 (m,2H), 2.60-2.74 (m,1H), 2.49-2.58 (m,2H), 1.82-1.91 (m,2H), 1.52-1.64 (m,2H); |
| **Example 12** | 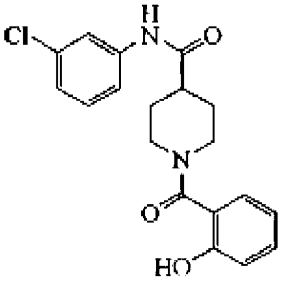 MAGLZ-II-12 | $^1$H NMR(DMSOd$_6$) δ (ppm): 10.15(s,1H),9.79(s,1H),7.835(t,1H,J=1.5Hz),7.42-7.48(m,1-H),7.32(t,1H,J=6Hz),7.18-7.26( m,1H),7. 06-7.13(m,2H),6.80-6.89(m,2H),2.69-2.89(m,4H), 2.55-2.65(m,1H),1.69-1.93(m,2H),1.49-1.67(m,2 H). |
| **Example 13** | 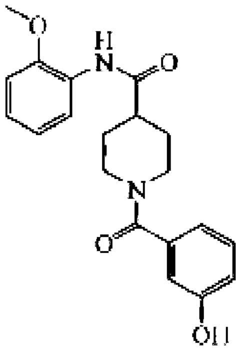 MAGLZ-II-13 | $^1$H NMR(DMSOd$_6$) δ (ppm): 9.64(s,1H),9.07(s,1H),7.87-8.00(m,1H), 7.24(t,1H,J=7.8Hz),6.99-7.13(m,2H),6.72-6.94(m, 4H),3.83(s,3H),2.62-3.16(m,4H),1.43-1.95(m,5H) |
| **Example 14** | 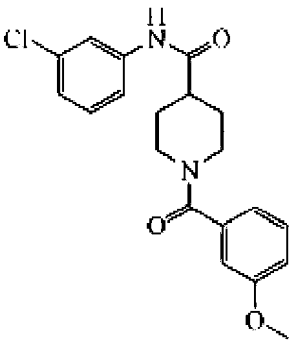 MAGLZ-II-14 | $^1$H NMR(DMSOd$_6$) δ (ppm): 10.12(s,1H), 7.83(t,1H,J=1.92Hz),7.43-7.50(m, 1H),7.30-7.39( m,2H),7.07-7.11(m,1H),7.00-7.04(m,1H), 6.91-6.96(m,2H),3.79(s,3H),2.67-3.30(m,4H),2.58 -2.67(m,1H),1.76-1.95(m,2H),1.53-1.66(m,2H). |

(continued)

| No. | Target product No. | Characterization data |
|---|---|---|
| **Example 15** | 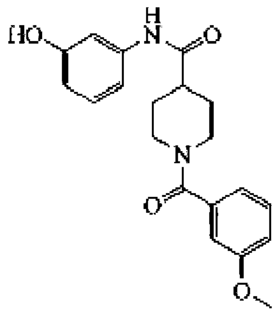 MAGLZ-II-15 | $^1H$ NMR(DMSOd$_6$) δ (ppm): 9.68(s,1H), 9.23(s,1H),7.32-7.42(m,1H),7.15-7.20(m,1H),6.90 -7.06(m,5H),6.40-6.48(m,1H),3.80(s,3H),2.68-3.2 0(m,4H),2.56-2.68(m,1H),1.76-1.79(m,2H),1.54-1 .66(m,2H). |
| **Example 16** | 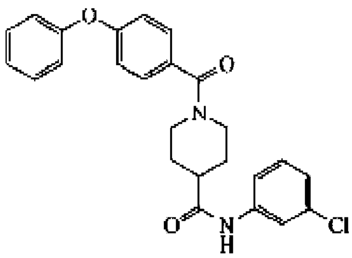 MAGLZ-II-16 | H),7.17-7.24(m,1H),7.07-7.12(m,3H),7.01-7.05(m $^1H$ NMR(DMSOd$_6$) δ (ppm): 10.13(s,1H), 7.79-7.88(m,1H),7.41-7.48(m,5H),7.29-7.36( m,1 H),7.17-7.24(m, 1H),7.07-7.12(m,3H),7.01-7.05(m ,3H),2.56-3.29(m,4H), 1.46-1.99(m,5H) |
| **Example 17** | 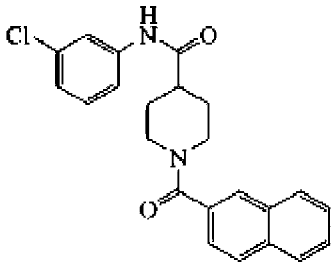 MAGLZ-II-17 | $^1H$ NMR(DMSOd$_6$) δ (ppm): 10.14(s,1H),7.97-8.04(m,4H),7.81-7.87(m,1H), 7.57-7.62(m,2H),7.44-7.54(m,2H),7.29-7.38 (m,1H),7.06-7.14(m,1H),3.65-4.66(m,2H),2.88-3. 22(m,2H),2.64-2.71(m,1H),1.62-1.97(m,4H). |
| **Example 18** | 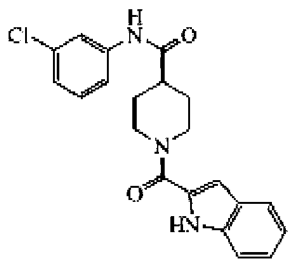 MAGLZ-II-18 | $^1H$ NMR(DMSOd$_6$) δ (ppm): 11.58(s,1H), 10.17(s,1H),7.82-7.88(m,1H),7.58-7.65(m,1H),7.4 0-7.50(m,2H),7.29-7.37(m,1H),7.15-7.23(m,1H),7 .01-7.13(-m,2H),6.78-6.82(m,1H),4.44-4.58(m,2H) ,3.00-3.26(m,2-H),2.66-2.74(m,1H),1.86-2.00(m,2 H),1.58-1.74(m,2H). |
| **Example 19** | 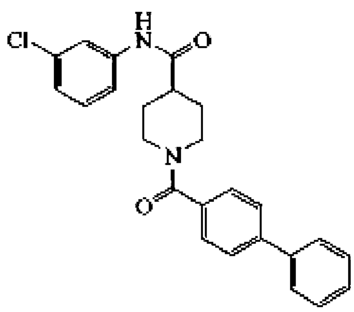 MAGLZ-II-19 | $^1H$ NMR(DMSOd$_6$) δ (ppm): 10.16(s,1H),7.83-7.86(m,1H),7.69-7.78(m,4H),7.4 7-7.52(m,4H),7.37-7.46(m,2H),7.33(t,1H,J=8.04), 7.07-7.13(m,1H),3.67-4.61(m,2H),2.89-3.16(m,2 H),2.61-2.69(m,1H),1.82-1.99(m,2H),1.56-1.69(m ,2H). |

(continued)

| No. | Target product No. | Characterization data |
|---|---|---|
| **Example 20** | 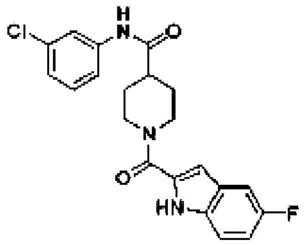 MAGLZ-II-18(a) | $[M+H]^+$401.2. |
| **Example 21** | 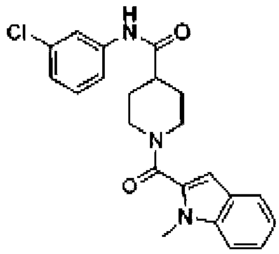 MAGLZ-II-18(c) | $^1$H NMR(300 MHz, DMSO-$d_6$) δ (ppm): 10.17(s,1H),7.83-7.87(t,1H),7.59-7.65(d,1H),7.50 -7.56(d,1H),7.45-7.49(m,1H),7.30-7.37(t,1H),7.23 -7.30(t,1H),7.11-7.14(m,1H),7.07-7.11(m,1H),6.6 7(s,1H),3.77(s,3H),2.63-2.76(m,2H),1.98-2.06(s,1 H),1.85-1.97(d,2H),1.57-1.73(m,2H),1.15-1.28(m, 2H). MS: $[M+Na]^+$: 418.2. |
| **Example 22** | 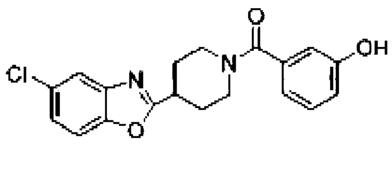 MAGLZ-II-30 | $^1$H NMR(300 MHz, DMSO-$d_6$) δ (ppm): 9.68 (s,1H), 7.83-7.84(d,1H), 7.73-7.75 (d,1H), 7.40-7.43 (m,1H), 7.21-7.25 (t,1H), 6.81-6.84 (m,1H), 6.78-6.80 (d,1H), 6.75-6.76 (m,1H), 4.40 (d,1H), 3.67 (s,1H), 3.21 (s,2H),3.09-3.15 (m,1H), 2.11-2.15 (m,2H), 1.75-1.78 (m,2H); $[M+Na]^+$379.1 |
| **Example 23** | 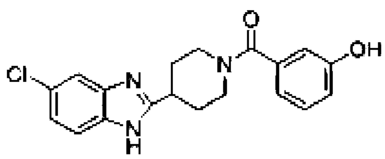 MAGLZ-II-31 | $^1$H NMR(300 MHz, DMSO-$d_6$) δ (ppm): 12.48 (s,1H), 9.73 (s,1H), 7.56-7.62(t,1H), 7.50-7.56 (d,1H), 7.24-7.29 (t,1H), 7.18-7.20 (m,1H), 6.80-6.87 (m,3H), 4.50 (d,1H), 3.71 (d,1H), 2.03-2.10 (m,2H), 1.71-1.81 (m,2H);3.21 (s,2H), 3.02-3.17 (m,1H),2.03-2.10 (m,2H), 1.71-1.81 (m,2H); $[M+H]^+$356.1 |

The target products synthesized in Examples 24-30 are described on the basis of the following structural formula:

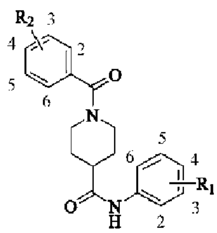
,

where, the $R_1$ is selected from hydroxy, F, Cl, Br, I, methyl, ethyl, propyl, and methoxy; $R_2$ is selected from hydroxy, F, Cl, Br, I, methyl, ethyl, propyl, methoxy, phenyl, and aryl substituted by hydroxyl, F, Cl, Br, I, methyl, ethyl, propyl, and methoxy. The number of $R_1$ and $R_2$ is 0, 1, 2, 3 or 4; the link mode between the $R_1$, the $R_2$ and nucleus is not limited to a single bond.

| Example | Compound | $R_1$ | $R_2$ | Characterization data |
|---|---|---|---|---|
| **Example 24** | MAGLZ-II-20 | 3-Cl | 4-OH | $^1$H NMR(300 MHz, DMSO-$d_6$) δ (ppm): 10.12 (s,1H), 9.83 (s,1H), 7.81-7.84 (m,1H), 7.42-7.49 (m,1H), 7.29-7.36 (m,1H), 7.22-7.28 (m,2H), 7.06-7.12 (m,1H), 6.77-6.83 (m,2H), 2.89-2.90 (m,2H), 2.73-2.74 (m,2H), 2.55-2.67 (m,1H), 1.77-1.89 (m,2H), 1.50-1.65 (m,2H);<br>$[M+H]^+$359.1 |
| **Example 25** | MAGLZ-II-21 | 3-C1 | 4-Cl | $^1$H NMR (300 MHz, DMSO-$d_6$) δ (ppm): 10.12 (s,1H), 7.81-7.84 (m,1H), 7.49-7.55 (m,2H), 7.41-7.48 (m,3H), 7.29-7.36 (m,1H), 7.07-7.12 (m,1H), 3.16-3.19 (m,2H), 2.79-3.11 (m,2H), 2.58-2.69 (m,1H), 1.71-1.95 (m,2H), 1.52-1.68 (m,2H);<br>$[M+H]^+$377.08 |
| **Example 26** | MAGLZ-II-22 | 3-Cl, and 4-Cl | 3-OH | $^1$H NMR (300 MHz, DMSO-$d_6$) δ (ppm): 10.24 (s,1H), 9.67 (s,1H),8.00-8.03 (m,1H), 7.47-7.58 (m,2H), 7.24 (t,1H, J = 7.5 Hz), 6.80-6.86 (m,1H), 6.74-6.80 (m,2H), 2.88-2.91 (m,2H), 2.69-2.74 (m,2H), 2.56-2.67 (m,1H), 1.72-1.93 (m,2H), 1.50-1.63 (m,2H);<br>$[M+H]^+$393.08 |
| **Example 27** | MAGLZ-II-23 | 3-Cl and 5-Cl | 3-OH | $^1$H NMR (300 MHz, DMSO-$d_6$) δ (ppm): 10.27 (s,1H), 9.67 (s,1H), 7.94-7.97 (m,1H), 7.67-7.70 (m,2H), 7.25-7.28 (m,1H), 6.72-6.85 (m,3H), 2.88-2.91 (m,2H), 2.73-2.74 (m,2H), 2.56-2.67 (m,1H), 1.73-1.92 (m,2H), 1.48-1.63 (m,2H);<br>$[M+H]^+$393.08 |
| **Example 28** | MAGLZ-II-24 | 3-Cl, and 4-Cl | 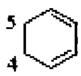 | $^1$H NMR (300 MHz, DMSO-$d_6$) δ (ppm): 10.24 (s,1H), 7.95-8.02 (m,5H), 7.57-7.60 (m,2H), 7.48-7.54 (m,3H), 2.89 (s,2H), 2.73 (s,2H), 2.61-2.65 (m,1H), 1.74-1.90 (m,2H), 1.57-1.67 (m,2H);<br>$[M+H]^+$ 427.09 |
| **Example 29** | MAGLZ-II-25 | 3-Cl and 5-Cl | 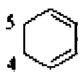 | $^1$H NMR (300 MHz, DMSO-$d_6$) δ (ppm): 10.28 (s,1H), 7.95-8.00 (m,4H), 7.68-7.69 (d,2H), 7.57-7.60 (m,2H), 7.49-7.52 (m,1H), 7.25-7.26 (t,1H), 2.89 (s,2H), 2.73 (s,2H), 2.59-2.63 (m,1H), 1.73-1.91 (m,2H), 1.58-1.65 (m,2H);<br>$[M+H]^+$ 427.09 |
| **Example 30** | MAGLZ-II-26 | 3-F | 3-OH | $^1$H NMR (300 MHz, DMSO-$d_6$) δ (ppm): 10.16 (s,1H), 9.69 (s,1H), 7.58-7.65 (m,1H), 7.27-7.38 (m,2H), 7.24 (t,1H, J = 7.5 Hz), 6.73-6.90 (m,4H), 2.71-3.12 (m,4H), 2.56-2.68 (m,1H), 1.73-1.93 (m,2H), 1.49-1.65 (m,2H);<br>$[M+H]^+$343.15 |
| **Example 31** | MAGLZ-II-27 | 3-F and 4-Cl | 3-OH | $^1$H NMR (300 MHz, DMSO-$d_6$) δ (ppm): 10.30 (s,1H), 9.76 (s,1H), 7.76-7.81 (m,1H), 7.47-7.53 (t,1H), 7.32-7.36 (m,1H), 7.21-7.27 (t,1H), 6.74-6.85 (m,3H), 2.66-2.89 (m,4H), 2.63-2.73 (m,1H), 1.76-1.85 (m,2H), 1.54-1.57 (m,2H);<br>$[M+H]^+$377.10 |
| **Example 32** | MAGLZ-II-29 | 3-Cl | 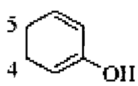 | $^1$H NMR (300 MHz, DMSO-$d_6$) δ (ppm): 10.13 (s,1H), 9.93 (s,1H), 7.82-7.87 (m,3H), 7.72-7.75 (d,1H), 7.44-7.47 (m,1H), 7.37-7.40(m,1H),7.29-7.34 (t,1H), 7.07-7.15 (m,3H), 2.64-3.02 (m,4H), 2.60-2.64 (m,1H), 1.82-1.85 (m,2H), 1.56-1.68 (m,2H);<br>$[M+Na]^+$431.12 |

(continued)

| Example | Compound | $R_1$ | $R_2$ | Characterization data |
|---|---|---|---|---|
| **Example 33** | MAGLZ-II-11 (a) | 3-Cl | 2-F and 5-OH | $^1$H NMR (300 MHz, DMSO-$d_6$) δ (ppm): 10.17 (s,1H), 9.68 (s,1H), 7.82 (s,1H), 7.43-7.45 (d,1H), 7.29-7.35 (t,1H), 7.06-7.09 (m,2H), 6.79-6.82 (m,1H), 6.67 (s,1H), 2.84-2.89 (d,2H), 2.68-2.73 (d,2H), 2.61-2.62 (m,1H), 1.76-1.93 (m,4H); $[M+Na]^+$399.1 |

**Example 34 Preparation of MAGLZ-II-11F:**

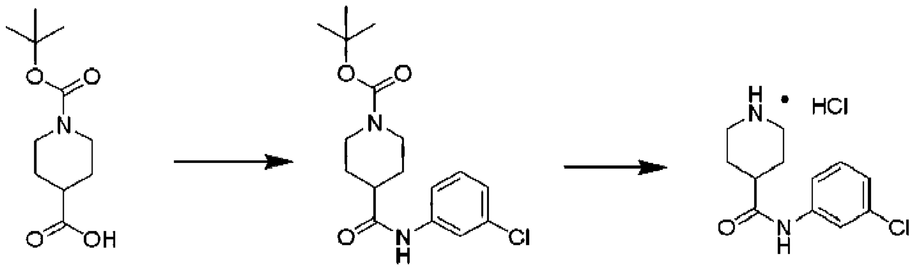

21h

22h

A raw material N-Boc-4-piperidine formic acid (20) (229.3 mg, 1 mmol) was dissolved into 10 mL $CH_2Cl_2$, DIPEA (0.35 mL, 2 mmol) and HATU (456 mg, 1.2 mmol) were added, and stirred for 10 min at room temperature, then 3-chloroaniline (0.21 mL, 2 mmol) was added and stirred for 6 h at room temperature, washed with water for three times, and washed by 10% diluted hydrochloric acid for three times, and dried by anhydrous $Na_2SO_4$, subjected to rotary evaporation to remove $CH_2Cl_2$, thus obtaining an intermediate 21 h without further purification.

The intermediate 21 h was dissolved into 10 mL EtOAc, and 4M HCl/EtOAc solution (2 mL) was added and stirred for 12 h at room temperature to separate out a white solid, then the white solid was subjected to suction filtration, and a filter cake was washed with EtOAc to obtain an intermediate 22 h, namely, a white solid (188.1 mg and yield: 68.4%).

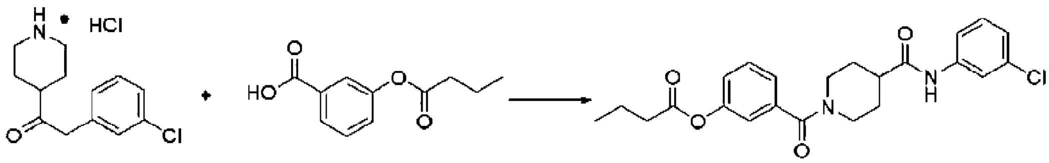

22h

24

MAGLZ-II-11F

The intermediate 24 (62.5 mg, 0.3 mmol) was dissolved into anhydrous DMF, HATU (136.8 mg, 0.36 mmol) and TEA (151.8 mg, 1.5 mmol) were added and stirred for 30 min at room temperature, then the hydrochloride intermediate 22 h (107.33 mg, 0.39 mmol) was added and stirred for 12 h at room temperature to stop the reaction; then a saturated salt solution was added and EtOAc was used for extraction for three times, organic phases were combined and washed for three times with a saturated salt solution and dried by anhydrous $Na_2SO_4$, then separated and purified by column chromatography (petroleum ether: ethyl acetate =1:1) to obtain a yellow solid (94.9 mg and yield: 74.1%).

Characterization data:

$^1$H NMR (300 MHz, $CDCl_3$) δ (ppm): 7.65 (s, 1H), 7.40-7.45 (t, 1H), 7.34-7.36 (d, 2H),7.23-7.28 (d, 1H), 7.14-7.16 (m, 2H), 7.07-7.10 (m, 1H), 2.96 (s, 1H), 2.88 (s, 1H), 2.52-2.58 (t, 2H), 2.04 (s, 1H), 1.74-1.86 (m, 2H), 1.59 (s, 4H), 1.24-1.28 (m, 3H), 1.02-1.07 (t, 3H); $[M+Na]^+$: 451.2.

**Example 35 Synthesis of MAGLZ-II-11J**

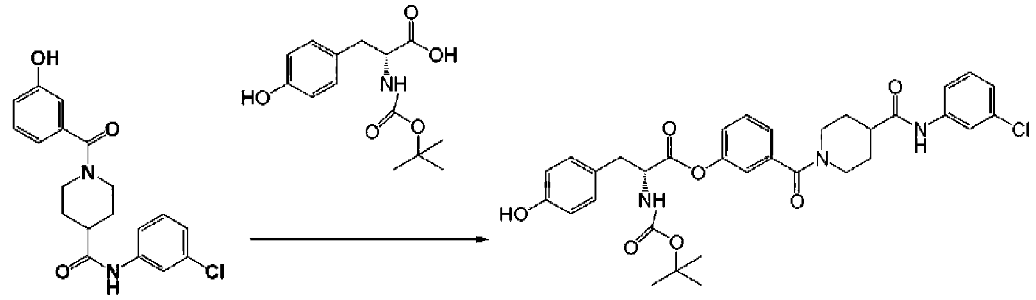

II-11 25

A raw material N-Boc-L-tyrosine (0.5 mmol, 140.5 mg) was dissolved into 10 mL anhydrous $CH_2Cl_2$, and the intermediate II-11 (179.5 mg, 0.5 mmol) and DMAP (0.1 mmol, 12.2 mg) were added, reacted and cooled to 0°C, then dissolved into DCC (0.55 mmol, 113.4 mg) of 3 mL anhydrous $CH_2Cl_2$, and stirred for 10 min, and reacted to room temperature, then the above materials were stirred for 12 h at room temperature; the reaction was incomplete, after the reaction was stopped, suction filtration was performed, and a filter liquor was rotary dried, and added with 20 mL $CH_2Cl_2$ and washed with a saturated salt solution for three times, and dried by anhydrous sodium sulfate. The above product was separated and purified by column chromatography (dichloromethane: methanol=100:1) to obtain an intermediate 25, namely, a white solid (52.6 mg, yield: 16.9%).

25 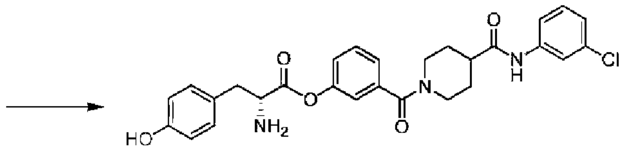

MAGLZ-II-11J

The intermediate 25 (37 mg) was dissolved into 10 mL EtOAc, and 4M HCl/EtOAc solution (2 mL) was added and stirred for 12 h at room temperature to separate out a white solid, then the white solid was subjected to suction filtration and a filter cake was washed with EtOAc, and the filter cake was added with 10 mL sodium bicarbonate and extracted with EtOAc for three times, then, organic phases were combined and washed with a saturated salt solution for three times and dried by anhydrous sodium sulfate, then subjected to rotary evaporation to remove EtOAc, thus obtaining a light white solid (12 mg and yield: 40%).

Characterization data of MAGLZ-II-11J: $^1$H NMR (300 MHz, DMSO-$d_6$) δ (ppm): 11.95 (s, 1H), 10.14 (s, 1H), 7.83 (s, 1H), 7.54-7.56 (d, 1H), 7.48-7.51 (t, 1H), 7.44-7.46 (d, 1H), 7.28-7.34 (m, 2H), 7.10-7.12 (m, 2H), 7.04-7.07 (d, 2H), 6.69-6.71 (d, 2H), 4.40-4.49 (m, 1H), 4.26-4.30 (t, 1H), 4.00-4.06 (m, 2H), 3.61 (s, 1H), 3.07-3.14 (d, 2H), 2.89-2.98 (d, 2H), 2.60-2.65 (m, 1H), 1.58-1.78 (m, 4H); $[M+Na]^+$544.17.

**Example 36: MAGL enzyme analysis**

A kit for screening the MAGL inhibitor from Cayman was used; according to the specification, an ethanol solution of 4-nitrophenylacetic acid served as a MAGL substrate, and the substrate had a final concentration of 236UM. 150 μL 1Xassay buffer, 10 μL recombinant human protein MAGL and 10 μL different concentrations of MAGL inhibitors (six points within 1 nM to 1000 nM) were added to per well of a 96-well plate. JZL195 (a positive control inhibitor) served as a positive control well, after incubation for 5 min at room temperature, per well was added with 10 μL MAGL substrate, and 86 wells were vibrated for 10 s and placed for 10 min at room temperature, then an absorbance was detected at 410 nm. 100% inhibition control wells (150 μL 1Xassay buffer, 10 μL MAGL and 10 μL DMSO were added per well of the 96-well plate, and three repeated wells were set) and background wells (160 μL 1Xassay buffer and 10 μL DMSO were added per well, and three repeated wells were set) were further set. Graphad Prism 5.0 was taken to calculate IC50 curves fitting each inhibitor. Specific values are shown in Table 2.

Table 2 Activity data

| Compound No. MAGLX | IC50 (μM) |
|---|---|
| MAGLZ-II-01 | 0.045 |
| MAGLZ-II-02 | 0.027 |

(continued)

| Compound No. MAGLX | IC50 ($\mu$M) |
|---|---|
| MAGLZ-II-03 | 0.749 |
| MAGLZ-II-04 | ND |
| MAGLZ-II-05 | 4.07 |
| MAGLZ-II-06 | 1.96 |
| MAGLZ-II-07 | 0.576 |
| MAGLZ-II-08 | 1.34 |
| MAGLZ-II-09 | 10.8 |
| MAGLZ-II-10 | 0.662 |
| MAGLZ-II-11 | 0.096 |
| MAGLZ-II-12 | ND |
| MAGLZ-II-13 | 0.926 |
| MAGLZ-II-14 | 0.333 |
| MAGLZ-II-15 | ND |
| MAGLZ-II-16 | 0.018 |
| MAGLZ-II-17 | 0.015 |
| MAGLZ-II-18 | 0.670 |
| MAGLZ-II-19 | 2.82 |
| MAGLZ-II-18 (c) | 0.0026 |
| MAGLZ-II-18 (a) | 2.15 |
| MAGLZ-II-20 | 9.92 |
| MAGLZ-II-21 | ND |
| MAGLZ-II-22 | 1.77 |
| MAGLZ-II-23 | 4.03 |
| MAGLZ-II-24 | 0.275 |
| MAGLZ-II-25 | 0.139 |
| MAGLZ-II-26 | 12.2 |
| MAGLZ-II-27 | 2.90 |
| MAGLZ-II-29 | 0.252 |
| MAGLZ-II-30 | 0.011 |
| MAGLZ-II-31 | ND |
| MAGLZ-II-11 (a) | 2.15 |
| MAGLZ-II-11F | 0.126 |
| MAGLZ-II-11J | 1.60 |

**Example 37 Tissue distribution test on the MAGL inhibitor of** the **present invention**

Detection reagent: MAGLZ-II-11, MAGLZ-II-16, and MAGLZ-II-17;

test object: mice;
administration dose: 16 mg/kg (formulation concentration: 8 mg/ml);
administration mode: subcutaneous injection;
sampling time: sampling was performed 1, 2, and 5 hours later after administration;

test tissues: brain, heart, liver, spleen, lung, kidney, pancreas, and blood;
experimental results indicate that:

3 test substances are distributed in the brain detected, where, the content of MAGLZ-II-16 in the brain is up to the highest, MAGLZ-II-17 is ranking the second, and MAGLZ-II-11 is the lowest (see Table 3).

Table 3 Tissue distribution diagram

| Time/h | MAGLZ-II-01 | MAGLZ-II-02 | MAGLZ-II-11 | MAGLZ-II-16 | MAGLZ-II-17 |
|---|---|---|---|---|---|
| 1.0 | 0 | 0 | 0 | 0 | 2.9 |
| 2.0 | 0.6 | 1.2 | 1.6 | 32.8 | 9.4 |
| 5.0 | 0.9 | 3.7 | 1.3 | 16.9 | 4.8 |

Example 38 Effect of the MAGL inhibitor of the present invention on reserpine-induced mice depression

1) Basic information

Species: ICR mouse

Microbiological level: SPF

Quantity and sex of the animals purchased: male: 75; female: 75

Quantity and sex of the animals used: male: 75; female: 75

Body weight: 13-18 g when accepted

2) Animal grouping

Group design: there were 50 pieces for each batch of animals and 3 batches in total; each batch of animals were randomly divided into 5 groups; low, middle and high-dose groups of the samples for test, blank control group and model group; a sample for test was tested per batch.

3) Test method

3.1 Animals were administrated via subcutaneous injection per day with doses of 4, 8 and 16 mg/kg for 6 d in total. 2 h later after the final administration, each animal was subcutaneously injected with 4 mg/kg reserpine except the blank control group; afterwards, indicators, such as, awakening, finger contact, fear, head touch, autonomic activity, upright and eyelid closure were determined 2 h later.

4) Statistical analysis

Data statistics and analysis were completed via a computer by SPSS 17.0 software and office 2016, and data statistics was inspected by Kruskal-Wallis. If the Kruskal-Wallis inspection result is significant (($P \leq 0.05$), multiple comparison inspection is further performed by a Mann-Whitney U nonparametric test method.

5) Results are shown in Tables 4A, 4B and 4C

**Table 4A**

| Group | Awakening | Finger contact | Head touch | Fear | Autonomic activity | Upright | Eyelid closure |
|---|---|---|---|---|---|---|---|
| Blank control | 4±0 | 4±0 | 4±0 | 4±0 | 4±0 | 4±0 | 4±0 |

(continued)

| Group | Awakening | Finger contact | Head touch | Fear | Autonomic activity | Upright | Eyelid closure |
|---|---|---|---|---|---|---|---|
| group | | | | | | | |
| Model group | 2.2±1.40** | 1.2±0.98** | 1.4±0.92** | 0.2±0.60** | 0±0** | 0±0** | 1.2±1.33** |
| MAGL-II-11-L | 3±1.61 | 1.6±1.50 | 1±1.34 | 0±0 | 0.8±1.60 | 0.8±1.60 | 1.2±0.98 |
| MAGL-II-11-M | 2.6±1.56 | 1.8±1.66 | 1.8±1.40 | 0±0 | 0.8±1.60 | 0.8±1.60 | 1.4±1.28 |
| MAGL-II-11-H | 3.2±1.33 | 2±1.79 | 2.6±1.56 | 0.2±0.60 | 1.8±1.66** | 1±1.61** | 2±1.26 |

Note: a comparison between the model group and the blank group: *$P$<0.05, **$P$<0.01; a comparison between the administration group and the model group: *$P$<0.05, **$P$<0.01.

**Table 4B**

| Group | Awakening | Finger contact | Head touch | Fear | Autonomic activity | Upright | Eyelid closure |
|---|---|---|---|---|---|---|---|
| Blank control group | 4±0 | 4±0 | 4±0 | 4±0 | 4±0 | 4±0 | 4±0 |
| Model group | 3.5±1.32** | 3.5±1.32** | 3.58±1.10** | 0.25±0.66** | 0.58±0.85** | 0±0** | 1.83±0.44** |
| MAGL-II-17-L | 4±0 | 4±0 | 3.75±0.66 | 3.5±1.32 | 0.5±0.87 | 0.25±0.6 6 | 2.25±0.66 |
| MAGL-II-17-M | 4±0 | 4±0 | 4±0 | 0±0 | 1.5±1.66 | 1±1.41 | 1±1.00 |
| MAGL-II-17-H | 4±0 | 4±0 | 3.75±0.66 | 0±0 | 0.25±0.66 | 0±0 | 0.25±0.66 |

Note: a comparison between the model group and the blank group: *$P$<0.05, **$P$<0.01; a comparison between the administration group and the model group: *$P$<0.05, **$P$<0.01.

**Table 4C**

| Group | Awakening | Finger contact | Head touch | Fear | Autonomic activity | Upright | Eyelid closure |
|---|---|---|---|---|---|---|---|
| Blank control group | 4±0 | 4±0 | 4±0 | 4±0 | 4±0 | 4±0 | 4±0 |
| Model group | 1.6±1.2** | 1.2±0.98** | 0±0** | 0±0** | 0.2±0.6** | 0±0** | 0±0** |
| MAGL-II-16-L | 2±0 | 1.4±0.92 | 0.4±0.8 | 0±0 | 0±0 | 0±0 | 0±0 |
| MAGL-II-16-M | 1.8±0.6 | 1.2±0.98 | 0.8±0.98* | 0±0 | 0.2±0.6 | 0±0 | 0±0 |
| MAGL-II-16-H | 2±0 | 1.6±0.8 | 0.8±0.98* | 0±0 | 0.6±0.6* | 0.4±1.2 | 0±0 |

Note: a comparison between the model group and the blank group: *$P$<0.05, **$P$<0.01; a comparison between the administration group and the model group: *$P$<0.05, **$P$<0.01.

5.1) MAGLZ-II-11:

Nonparametric test

Each indicator of the Kruskal-Wallis test has a statistic difference (P<0.05);

Comparison of difference between groups

Mann-Whitney U test

The comparison between the model group and the blank control group shows a statistic difference (P<0.05), indicating a good modeling result; the comparison between the low/middle/high-dose group and the model group shows

no statistic difference ($P>0.05$); the comparison between the high-dose group and the model control group (autonomic activity and upright) shows a statistic difference ($P<0.01$, $P<0.05$); the variation trend of the indicator is consistent with the blank control.

5.2) MAGLZ-II-17: each indicator of the Kruskal-Wallis test has a statistic difference ($P<0.05$); Mann-Whitney U test

The comparison between the model group and the blank control group shows a statistic difference ($P<0.05$), indicating a good modeling result;

the comparison between the low-dose group and the model group shows no statistic difference ($P>0.05$);

the comparison between the middle-dose group (eyelid closure) and the model group shows a statistic difference ($P<0.05$); but the mean value is less than that of the model group; and other indicators show no statistic difference ($P>0.05$);

the comparison between the high-dose group (eyelid closure) and the model group shows a statistic difference ($P<0.01$); but the mean value is less than that of the model group; and other indicators show no statistic difference ($P>0.05$);

5.3) MAGLZ-II-16: each indicator of the Kruskal-Wallis test has a statistic difference ($P<0.05$); Mann-Whitney U test

The comparison between the model group and the blank control group shows a statistic difference ($P<0.05$), indicating a good modeling result;

the comparison between the low-dose group and the model group shows no statistic difference ($P>0.05$);

the comparison between the middle-dose group (head touch) and the model group shows a statistic difference ($P<0.05$); but the mean value is greater than that of the model group and has a trend change the same as that of the blank control group; and other indicators show no statistic difference ($P>0.05$);

the comparison between the high-dose group (head touch and autonomic activity) and the model group shows a statistic difference ($P<0.05$); but the mean value is greater than that of the model group and has a trend change the same as that of the blank control group; and other indicators show no statistic difference ($P>0.05$).

6) Conclusion

6.1) MAGLZ-II-11 has a certain effect on reserpine-induced depression under this test condition.

6.2) MAGLZ-II-16 has a certain effect on reserpine-induced depression under this test condition.

6.3) MAGLZ-II-17 may enhance the effect on reserpine-induced depression under this test condition.

Example 39 Effect of the MAGL inhibitor of the present invention on mice forced swimming test and tail suspension model

1 Materials and methods

1.1 Experimental animal

Male ICR mice having a body weight of 18-22 g. The animals were active on a schedule of normal illumination for 12 h and in the dark for 12 h. Environmental temperature and relative humidity were relatively kept for $22\pm1°C$ and $55\pm5\%$.

1.2 Experimental method

1.2.1 Animal grouping and administration method

There were 7 groups of laboratory mice, 10 pieces each group, set as a control group (normal saline), a positive control group (fluoxetine hydrochloride capsule, 5 mg/kg), 5 MAGLZ-II series of administration treatment groups: MAGLZ-II-06, MAGLZ-II-07, MAGLZ-II-11, MAGLZ-II-17 and MAGLZ-II-18, respectively, with an administration concentration of 5 mg/kg.

All medicaments were used once per day via intragastric administration with a dose of 20 mL/kg (body weight), lasting for 10 d. 1 h later after the final administration, the detection of animal behaviors was started.

1.2.2 Mice forced swimming test

Mice forced swimming test was performed according to the method established by Porsolt (Porsolt R D. Behavioural despair in mice: A primary screening test for antidepressants[J]. Arch. Int. Pharmacolodyn. 1977, 229). The mice were separately subjected to forced swimming in an open cylindrical container (diameter: 10 cm and height: 25 cm); the container had a water temperature of 25±1°C and a water depth of 19 cm; the total time that each animal kept still in the course of 6 min was recorded as immobility time (unit: s). Each mouse was judged as still when stopped struggling, kept suspension in water and only made necessary movement to keep the head above the water. Decrease of the immobility time indicates that there is an anti-depression effect.

1.2.3 Mice tail suspension test

The tail suspension test (TST) was performed by the method established by Porsolt, et al. (Porsolt R D, Le Pichon M, Jalfre M. Depression: a new animal model sensitive to antidepressant treatments[J]. Nature, 1977, 266(5604):730-732). Mice were respectively suspended on the tail portions with tail suspension clamps at a distance of 10 mm away from the tail portion of the box (250 mm×250 mm×300 mm) and a distance of 5 cm where the head of each mouse was away from the bottom portion. Noise was tested in a dark room having minimum background, and each mouse was suspended for 6 min, and an immobility time was recorded at intervals in the final 4 min. Judgment standard: the mice stopped struggling and kept still completely.

1.2.4 Data processing

All data are shown by $\bar{x}$±SD, and the comparison among groups is analyzed by one-way variance.

2 Results

2.1 Influences of MAGLZ-II series of compounds on forced swimming test

The test for the influences of MAGLZ-II series of compounds on mice forced swimming test indicates that the positive control medicament, fluoxertine hydrochloride and MAGLZ-II series of compounds may obviously shorten the accumulated immobility time (P<0.05) of mice forced swimming test (Table 5).

Table 5 Influences of the MAGLZ-II series of compounds on mice forced swimming test

| Group | Dose (mg/kg) | Number animals (pcs.) | of Accumulated immobility time (S) |
|---|---|---|---|
| Control group | - | 10 | 136.84±46.69 |
| Fluoxetine hydrochloride capsule | 5 | 10 | 92.64±29.17** |
| MAGLZ-II-06 | 5 | 10 | 89.23±46.37* |
| MAGLZ-II-07 | 5 | 10 | 91.31±41.85* |
| MAGLZ-II-11 | 5 | 10 | 73.57±27.28** |
| MAGLZ-II-17 | 5 | 10 | 79.38±41.67** |
| MAGLZ-II-18 | 5 | 10 | 81.71±38.12** |
| Note: compared with the control group, *P<0.05 and **P<0.01. | | | |

2.2 Influences of the MAGLZ-II series of compounds on mice tail suspension test

Compared with the control group, the MAGLZ-II series of compounds may obviously shorten the accumulated immobility time (P<0.05) of tail suspension mice (Table 6).

Table 6 Influences of the MAGLZ-II series of compounds on mice tail suspension behaviors

| Group | Dose (mg/kg) | Number of animals (pcs.) | Accumulated immobility time (S) |
|---|---|---|---|
| Control group | - | 10 | 132.38±44.51 |
| Fluoxetine hydrochloride capsule | 5 | 10 | 92.19±23.63** |
| MAGLZ-II-06 | 5 | 10 | 90.73±30.51** |
| MAGLZ-II-07 | 5 | 10 | 89.66±31.52* |

(continued)

| Group | Dose (mg/kg) | Number of animals (pcs.) | Accumulated immobility time (S) |
|---|---|---|---|
| MAGLZ-II-11 | 5 | 10 | 72.81±23.49** |
| MAGLZ-II-17 | 5 | 10 | 89.87±29.68** |
| MAGLZ-II-18 | 5 | 10 | 79.68±25.72** |
| Note: compared with the control group, *$P$<0.05 and **$P$<0.01. | | | |

3 Discussion

Mice forced swimming test and tail suspension test are comparatively common and classical depression models for animals. Despair, behavior distortion and other conditions of the mice reflected by the model are significant features of in vitro depression simulation; meanwhile, the mice are more sensitive to antidepressants and easy to operate. Therefore, the two models are common screening models for antidepressants. Such two classical depression models for animals are used in this study to further evaluate and verify the anti-depression activity of the MAGLZ-II series of compounds. The results indicate that the MAGLZ-II series of compounds may significantly shorten the accumulated immobility time of the mice forced swimming test and tail suspension test, showing that the MAGLZ-II series of compounds have a specific antidepression effect.

**Example 40 Influences of the MAGL inhibitor of the present invention on the mice writhing times caused by acetic acid**

**1. Animal grouping and administration**

200 male ICR mice (20±2) g were divided into 20 groups randomly according to body weight, 10 pieces for each group. The specific grouping conditions are as follows: model group: an equal volume of 0.5% sodium carboxymethylcellulose, ig

positive control group: 10 mg/kg diclofenac sodium, ig

MAGLZ-II-11 low-dose group: 7.5 mg/kg, ig

MAGLZ-II-11 middle-dose group: 15 mg/kg, ig

MAGLZ-II-11 high-dose group: 30 mg/kg, ig

MAGLZ-II-11a low-dose group: 7.5 mg/kg, ig

MAGLZ-II-11a middle-dose group: 15 mg/kg, ig

MAGLZ-II-11a high-dose group: 30 mg/kg, ig

MAGLZ-II-18a low-dose group: 7.5 mg/kg, ig

MAGLZ-II-18a middle-dose group: 15 mg/kg, ig

MAGLZ-II-18a high-dose group: 30 mg/kg, ig

MAGLZ-II-18 low-dose group: 7.5 mg/kg, ig

MAGLZ-II-18 middle-dose group: 15 mg/kg, ig

MAGLZ-II-18 high-dose group: 30 mg/kg, ig

MAGLZ-II-18c low-dose group: 7.5 mg/kg, ig

MAGLZ-II-18c middle-dose group: 15 mg/kg, ig

MAGLZ-II-18c high-dose group: 30 mg/kg, ig

MAGLZ-II-10 low-dose group: 7.5 mg/kg, ig

MAGLZ-II-10 middle-dose group: 15 mg/kg, ig

MAGLZ-II-10 high-dose group: 30 mg/kg, ig

Each compound was prepared into a suspension by 0.5% sodium carboxymethylcellulose respectively, and then set according to the dose, and each administration group was administered intragastrically; the model group was administered intragastrically an equal volume of 0.5% sodium carboxymethylcellulose.

**2. Experimental method and data processing**

Mice were intraperitoneally injected with acetic acid to cause a large area and more lasting painful stimuli in deep enterocoelia, causing mice to produce writhing responses. After being administered for 1 h, each dose group was intraperitoneally injected with 0.7% acetic acid-normal saline solution (0.1 ml/10 g).

The time (pain latent time) that the mice started to appear writhing after being intraperitoneally injected with acetic acid was recorded.

The writhing response times of each mouse within 20 min were recorded after injecting acetic acid to cause pain, and then, the writhing inhibition ratio of each administration group was calculated.

Writhing inhibition ratio=[(writhing times of the control group - writhing times of the administration group)/writhing times of the control group] $\times$ 100%

Experimental data is shown by $\bar{x} \pm s$ and subjected to variance analysis by SPSS15.0 software.

**3. Experimental results**

Experimental results are shown in Table 7.

Table 7 Influences of the MAGL inhibitor of the present invention on the mice writhing times caused by acetic acid

| Group | Writhing times | Inhibition ratio (%) | Latent time (s) |
|---|---|---|---|
| Model group | 39.3±12.9 | - | 226.5±38.2 |
| Positive medicament group | 24.5±12.9* | 37.7 | 358.5±172.7* |
| MAGLZ-II-18a low-dose group | 24.2±8.5** | 38.4 | 355.5±136.5* |
| MAGLZ-II-18a middle-dose group | 20.8±12.5** | 47.1 | 380.7±130.7** |
| MAGLZ-II-18a high-dose group | 17.8±11.1*** | 54.7 | 422.4±137.9*** |
| MAGLZ-II-11 low-dose group | 23.4±11.7** | 40.5 | 379.7±129.3** |
| MAGLZ-II-11 middle-dose group | 17.5±9.6*** | 55.5 | 408.4±128.2*** |
| MAGLZ-II-11 high-dose group | 13.6±13.1*** | 65.4 | 467.8±175.6*** |
| MAGLZ-II-11a low-dose group | 22.6±12.0** | 42.5 | 401.2±168.3** |
| MAGLZ-II-11a middle-dose group | 18.6±14.6** | 52.7 | 438.9±230.0** |
| MAGLZ-II-11a high-dose group | 12.5±9.4*** | 68.2 | 488.8±172.0*** |
| MAGLZ-II-18 low-dose group | 25.0±13.5* | 36.4 | 318.7±120.2* |
| MAGLZ-II-18 middle-dose group | 21.8±12.4** | 44.5 | 381.4±127.1** |
| MAGLZ-II-18 high-dose group | 15.8±15.3** | 59.8 | 410.9±186.5** |
| MAGLZ-II-18c low-dose group | 28.3±11.9 | 28.0 | 263.5±77.7 |
| MAGLZ-II-18c middle-dose group | 24.9±12.2* | 36.6 | 312.7±90.9* |
| MAGLZ-II-18c high-dose group | 19.9±12.2** | 49.4 | 373.6±114.0** |
| MAGLZ-II-10 low-dose group | 34.0±9.1 | 13.5 | 235.4±59.6 |
| MAGLZ-II-10 middle-dose group | 32.8±11.6 | 16.5 | 246.2±69.5 |

(continued)

| Group | Writhing times | Inhibition ratio (%) | Latent time (s) |
|---|---|---|---|
| MAGLZ-II-10 high-dose group | 24.8±11.1* | 36.9 | 306.4±90.2* |

Compared with the model group, *P<0.05, **P<0.01 and ***P<0.001.

It can be seen from the experimental results shown in Table 1 that compared with the model group, the MAGLZ-II-11, MAGLZ-II-11a, MAGLZ-II-18a and MAGLZ-II-18c of the present invention may significantly decrease the mice writhing times caused by acetic acid and extend latent time.

**Example 41 Influences of the MAGL inhibitor of the present invention on the mice pain response induced by hot plate**

Female ICR mice (20±2) g were placed on an intelligent hot plate at 55±0.5 °C, and latency (s) that the plantar of mice touched the hot plate until the occurrence of a hind paws licking response served as a pain threshold indicator; the mice (response latency <5 s or >30 s) or jumping mice were eliminated.

Grouping and administration were the same as those in Example 40.

The mice were continuously administered intragastrically for 7 consecutive days; the pain threshold of each group of mice was respectively determined once at 30, 60, 90 and 120 min after the final administration; the pain threshold greater than 60 s was calculated by 60 s.

Experimental results are shown in Table 8.

Table 8 Influences of the MAGL inhibitor of the present invention on the pain threshold of mice pain response induced by hot plate

| | Pain thresholds of the mice at different periods of time after administration (s) | | | |
|---|---|---|---|---|
| Group | 30 min | 60 min | 90 min | 120 min |
| Model group | 15.63±7.32 | 16.85±7.46 | 15.50±6.11 | 15.38±6.93 |
| Positive medicament group | 22.32±6.76* | 24.34±8.19* | 22.64±6.48* | 22.52±6.91* |
| MAGLZ-II-11 low-dose group | 37.92±8.83*** | 44.14±7.56*** | 45.93±8.38*** | 43.80±6.12*** |
| MAGLZ-II-11 middle-dose group | 40.33±8.25*** | 44.87±5.67*** | 46.41±5.36*** | 43.44±5.91*** |
| MAGLZ-II-11 high-dose group | 41.43±5.15*** | 46.16±6.63*** | 47.22±5.98*** | 45.49±6.31*** |
| MAGLZ-II-11a low-dose group | 36.18±8.50*** | 43.17±7.43*** | 42.83±7.34*** | 42.16±7.05*** |
| MAGLZ-II-11a middle-dose group | 38.16±7.00*** | 45.47±7.03*** | 43.71±5.87*** | 42.34±7.04** |
| MAGLZ-II-11a high-dose group | 39.45±6.75*** | 46.56±6.35*** | 45.42±6.84*** | 43.73±5.68*** |
| MAGLZ-II-18a low-dose group | 34.62±6.65*** | 40.29±6.73*** | 43.98±6.55*** | 43.56±6.01*** |
| MAGLZ-II-18a middle-dose group | 37.51±6.07*** | 42.42±5.90*** | 45.86±7.09*** | 44.38±5.44*** |
| MAGLZ-II-18a high-dose group | 40.86±7.17*** | 46.29±5.27*** | 48.70±5.73*** | 47.04±5.54*** |
| MAGLZ-II-18 low-dose group | 26.33±7.52** | 26.74±7.05** | 27.16±7.32** | 26.64±10.03** |
| MAGLZ-II-18 middle-dose group | 26.41±7.53** | 27.09±7.53** | 27.58±7.71** | 27.38±9.97** |
| MAGLZ-II-18 high-dose group | 26.73±7.37** | 27.22±7.29** | 27.86±7.93** | 27.62±10.01** |
| MAGLZ-II-18c low-dose group | 23.98±6.89* | 24.75±6.91* | 25.16±8.87* | 24.28±7.17* |
| MAGLZ-II-18c middle-dose group | 26.82±7.99** | 28.52±6.18** | 28.66±8.73** | 27.88±7.74** |
| MAGLZ-II-18c high-dose group | 29.23±7.95** | 30.24±7.80** | 30.66±8.35** | 29.87±7.43** |
| MAGLZ-II-10 low-dose group | 16.37±6.00 | 17.93±5.75 | 19.24±4.72 | 19.14±4.63 |
| MAGLZ-II-10 middle-dose group | 16.67±5.97 | 18.79±5.95 | 22.43±5.31 | 20.60±4.64 |
| MAGLZ-II-10 high-dose group | 17.73±6.20 | 19.77±6.73 | 25.25±7.01* | 24.90+5.73* |

Compared with the model group, *P<0.05, **P<0.01 and ***P<0.001.

Compared with the model group, the MAGLZ-II-11, MAGLZ-II-11a, MAGLZ-II-18a, MAGLZ-II-18, and MAGLZ-II-18c of the present invention may significantly extend pain thresholds of the thermal-stimulus mice.

**Example 42 Influences of the MAGL inhibitor of the present invention on a pain threshold of the mice with inflammatory pain**

Male SD mice (180±20) g were injected with a complete Freund's adjuvant (50 μL/piece) at the right rear plantar of mice, and mice in the control group were injected with the corresponding volume of 0.5% sodium carboxymethylcellulose to establish a paw edema inflammatory pain model. After modeling, the mice were administered for 7 d continuously, and administered intragastrically once per day. Pain thresholds (s) of the mice were respectively determined on the 1st, 3rd and 7th day before administration and after modeling at different periods of time.

Grouping and administration were the same as those of Example 40.

Table 9 Influences of the MAGL inhibitor of the present invention on the pain thresholds of the mice with inflammatory pain

| Group | Pain thresholds of the mice at different periods of time after administration (s) | | | |
|---|---|---|---|---|
| | 0 d | 1 d | 3 d | 7 d |
| Control group | 12.67±2.52 | 13.02±3.03 | 12.91±2.81 | 12.88±2.88 |
| Model group | 12.56±2.50 | 5.49±0.94### | 7.06±0.63### | 8.27±0.57### |
| Positive medicament group | 12.71±2.55 | 6.38±0.91* | 8.05±1.23* | 9.00±0.911* |
| MAGLZ-II-11 low-dose group | 12.94±2.41 | 7.55±1.29*** | 9.00±0.73 | 10.26±1.08*** |
| MAGLZ-II-11 middle-dose group | 12.80±2.27 | 7.73±1.10*** | 9.47±0.79*** | 10.93±1.62*** |
| MAGLZ-II-11 high-dose group | 12.88±2.27 | 7.99±0.90*** | 9.88±1.34*** | 11.26±1.82*** |
| MAGLZ-II-11a low-dose group | 12.96±2.44 | 7.38±1.19*** | 8.87±0.74*** | 9.91±1.05*** |
| MAGLZ-II-11a middle-dose group | 13.03±2.35 | 7.58±0.84*** | 9.30±0.75*** | 10.66±1.30*** |
| MAGLZ-II-11a high-dose group | 12.97±2.32 | 7.83±1.01*** | 9.55±1.58*** | 11.00±161*** |
| MAGLZ-II-18a low-dose group | 12.73±2.43 | 6.97±0.87** | 8.44±1.03** | 9.56±0.92** |
| MAGLZ-II-18a middle-dose group | 12.86±2.25 | 7.26±0.70*** | 9.01±0.68*** | 10.37±1.49*** |
| MAGLZ-II-18a high-dose group | 12.85±2.26 | 7.49±0.79*** | 9.35±1.2*** | 10.37±1.59*** |
| MAGLZ-II-18 low-dose group | 12.73±2.43* | 6.39±0.91* | 8.12±1.29* | 9.16±1.17* |
| MAGLZ-II-18 middle-dose group | 12.86±2.25** | 6.62±0.69** | 8.24±0.73** | 9.51±1.15** |
| MAGLZ-II-18 high-dose group | 12.85±2.26** | 6.95±0.85** | 8.49±1.15** | 9.62±0.93** |
| MAGLZ-II-18c low-dose group | 12.75±2.46* | 6.36±0.89* | 7.98±1.21* | 8.95±0.67* |
| MAGLZ-II-18c middle-dose group | 12.86±2.07* | 6.41±0.91* | 8.10±1.30* | 8.99±0.92* |
| MAGLZ-II-18c high-dose group | 12.85±2.09* | 6.44±0.92* | 8.17±1.14* | 9.05±0.84* |
| MAGLZ-II-10 low-dose group | 12.71±2.47 | 5.59±0.89 | 7.15±0.59 | 8.33±0.57 |
| MAGLZ-II-10 middle-dose group | 12.77±2.48 | 5.62±0.84 | 7.18±0.55 | 8.36±0.59 |
| MAGLZ-II-10 high-dose group | 12.93±2.26 | 5.67±0.93 | 7.95±0.66* | 8.93±0.61* |

Compared with the model group, *P<0.05, **P<0.01 and ***P<0.001.

Compared with the model group, the MAGLZ-II-11, MAGLZ-II-11a, MAGLZ-II-18a, MAGLZ-II-18 and MAGLZ-II-18c of the present invention may significantly extend the pain threshold of the mice with inflammatory pain.

**Example 43 Therapeutical effect of the MAGL inhibitor of the present invention on irritable bowel syndrome rats induced by early maternal separation**

Irritable Bowel Syndrome (IBS) is a kind of common functional disease of stomach and intestine, manifested as chronic abdominal pain, abdominal discomfort and change of bowel evacuation habit, and no obvious intestinal lesion. This

experiment is mainly used to discuss the therapeutical effect of the MAGL inhibitor of the present invention on irritable bowel syndrome rats induced by early maternal separation.

**1. Animal grouping and administration**

200 SD rats (20±2) g were divided into 20 groups randomly according to body weight, 10 pieces for each group. The specific grouping conditions are as follows:

blank group: an equal volume of 0.5% sodium carboxymethylcellulose, ig

model group: an equal volume of 0.5% sodium carboxymethylcellulose, ig

MAGLZ-II-11 low-dose group: 7.5 mg/kg, ig

MAGLZ-II-11 middle-dose group: 15 mg/kg, ig

MAGLZ-II-11 high-dose group: 30 mg/kg, ig

MAGLZ-II-11a low-dose group: 7.5 mg/kg, ig

MAGLZ-II-11a middle-dose group: 15 mg/kg, ig

MAGLZ-II-11a high-dose group: 30 mg/kg, ig

MAGLZ-II-18a low-dose group: 7.5 mg/kg, ig

MAGLZ-II-18a middle-dose group: 15 mg/kg, ig

MAGLZ-II-18a high-dose group: 30 mg/kg, ig

MAGLZ-II-18 low-dose group: 7.5 mg/kg, ig

MAGLZ-II-18 middle-dose group: 15 mg/kg, ig

MAGLZ-II-18 high-dose group: 30 mg/kg, ig

MAGLZ-II-18c low-dose group: 7.5 mg/kg, ig

MAGLZ-II-18c middle-dose group: 15 mg/kg, ig

MAGLZ-II-18c high-dose group: 30 mg/kg, ig

MAGLZ-II-10 low-dose group: 7.5 mg/kg, ig

MAGLZ-II-10 middle-dose group: 15 mg/kg, ig

MAGLZ-II-10 high-dose group: 30 mg/kg, ig

Each compound was prepared into a suspension by 0.5% sodium carboxymethylcellulose respectively, and then set according to the dose, and each administration group was administered intragastrically; the model group was administered intragastrically an equal volume of 0.5% sodium carboxymethylcellulose.

**2. Experimental method and data processing**

The newborn SD male rats were separated from the mother rat in lactation period for 3 h every day on the 2nd to the 21st day after the birth of the newborn SD rats, namely, the mother rat in lactation period was taken out of the cage at 8:30 a.m., and then the newborn rats were removed from the initial cage and put to another separate cage, then the cage was transferred to an adjacent room, then the newborn rats were returned to the initial cage 3 h later, namely, at 11:30 such

that the newborn rats and the mother rat got together; the newborn rats were weaned on the 22nd day after birth and separated from the initial cage on the 30th day, grouped on the 60th day, and administered, 20 d in total.

Intragastric administration was performed within the administration period once a day with continuous administration for 20 d.

End point of the experiment: dissection was performed after finishing the corresponding test indicators of the experiment.

Test indicators: abdominal withdrawal reflex (AWR); test method: rats were fasted 18 h before the experiment and drank water freely, and narcotized with diethyl ether; then an air sac coated with paraffin oil was inserted into the rats' colorectum for 4.0 cm, and a catheter outside the anus was fixed at the root of the rat tail with an adhesive tape, and connected with an injection syringe and a sphygmomanometer via three links. The rats were put to an organic glass inspection box (20 cm×12 cm×9 cm), after the rats were awake and fully adapted to the environment for 30 min, the experiment was started. 20, 40 and 60 mm Hg of pressure were respectively applied on the rats, and continuous expansion was performed for 20 s each time with a stimulus interval of 4 min; each pressure was performed for 3 times repeatedly for AWR scoring. AWR scoring was performed by a single blind method; scoring standard: 0 points: no obvious behavior change; 1 point: simple head movement only; 2 points: abdominal muscles started to shrink but are not separated from the table top; 3 points: abdominal muscles obviously shrank to flatten or inferior abdominal wall was separated from the table top; 4 points: abdominal wall hunched up or body/pelvis hunched up.

At the end of the experiment, the animals were dissected and weighed, and narcotized to take blood, then the content of 5-HT in serum was determined by high performance liquid chromatography. Cerebrum was taken on an ice and precooled and cleaned slightly with 0.5% sodium carboxymethylcellulose, and then sucked dry with a filter paper, and placed into a cryogenic tube, and preserved at -20°C for further use, then the content of 5-HT in brain tissues was detected by high performance liquid chromatography.

Colon histological observation: after the experiment on colectasia, distal colon (5-6 cm away from the anus) of the rat was taken and subjected to conventional HE dyeing to observe the inflammation and damage conditions of the colon wall.

Data is presented by a mean±SD, and the difference among groups is subjected to statistics with t tests or a one-way ANOVA method, and $P<0.05$ shows a significant difference.

**3. Experimental results**

Experimental results are shown in Table 10.

Table 10 Influences of the MAGL inhibitor of the present invention on the AWR scoring and 5-HT of the IBS rats

| Group | Quantity | AWR scoring 20 mm Hg | 40 mm Hg | 60 mm Hg | Colon (μg/mg) 5-HT | Serum (μg/ml 5-HT |
|---|---|---|---|---|---|---|
| Blank group | 10 | 1.93±0.43 | 2.78±0.35 | 3.29±0.25 | 11.78±1.78 | 28.55±4.03 |
| Model group | 10 | 2.85±0.28### | 3.53±0.22### | 3.86±0.13### | 19.76±2.52### | 38.73±3.42### |
| MAGLZ-II-18c Low-dose group | 10 | 2.28±0.58* | 3.20±0.43* | 3.61±0.24** | 16.84±2.87* | 34.2±5.37* |
| MAGLZ-II-18c Middle-dose group | 10 | 1.79±0.23*** | 2.87±0.65** | 3.27±0.51** | 14.99±2.90** | 31.88±4.40** |
| MAGLZ-II-18 c High-dose group | 10 | 1.38±0.51*** | 2.20±0.57*** | 2.96±0.70*** | 14.34±2.40*** | 29.03±5.29*** |
| MAGLZ-II-11 a Low-dose group | 10 | 2.12±0.36*** | 3.02±0.53* | 3.58±0.26** | 16.33±2.78** | 31.76±5.42** |
| MAGLZ-II-11 a Middle-dose group | 10 | 1.51±0.43*** | 2.52±0.38*** | 3.27±0.48*** | 14.79±2.73*** | 29.89±5.34*** |
| MAGLZ-II-11 a High-dose group | 10 | 1.23±0.50*** | 2.13±0.48*** | 2.87±0.46*** | 12.65±3.36*** | 24.05±6.17*** |
| MAGLZ-II-10 Low-dose group | 10 | 2.87±0.39 | 3.43±0.30 | 3.79±0.20 | 21.44±2.72 | 36.32±3.14 |
| MAGLZ-II-10 Middle-dose group | 10 | 2.83±0.47 | 3.56±0.22 | 3.80±0.17 | 20.97±2.24 | 38.65±2.68 |

(continued)

| Group | Quantity | AWR scoring 20 mm Hg | 40 mm Hg | 60 mm Hg | Colon (μg/mg) 5-HT | Serum (μg/ml 5-HT |
|---|---|---|---|---|---|---|
| MAGLZ-II-10 High-dose group | 10 | 2.89±0.46 | 3.21±0.35* | 3.65±0.17* | 18.66±2.38* | 33.09±2.3* |
| MAGLZ-II-11 Low-dose group | 10 | 2.50±0.38* | 3.00±0.49** | 3.60±0.22** | 16.24±2.34** | 31.75±5.33** |
| MAGLZ-II-11 Middle-dose group | 10 | 2.02±0.57** | 2.42±0.41*** | 3.03±0.37*** | 14.33±2.10*** | 29.47±4.91*** |
| MAGLZ-II-11 High-dose group | 10 | 1.39±0.30*** | 1.92±0.33*** | 2.86±0.26*** | 12.00±2.93*** | 23.82±6.34*** |
| MAGLZ-II-18 Low-dose group | 10 | 2.59±0.33 | 3.26±0.39 | 3.65±0.23* | 21.91±2.46 | 35.73±3.32 |
| MAGLZ-II-18 Middle-dose group | 10 | 2.29±0.36** | 3.01±0.40** | 3.3±0.45** | 14.89±3.19** | 29.12±7.05** |
| MAGLZ-II-18 High-dose group | 10 | 1.87±0.36*** | 2.6±0.54*** | 3.09±0.34*** | 14.33±2.10*** | 30.86±5.28*** |
| MAGLZ-II-18 a Low-dose group | 10 | 2.58±0.26* | 3.06±0.38 | 3.50±0.29** | 16.43±2.15** | 32.51±5.01** |
| MAGLZ-II-18 a Middle-dose group | 10 | 2.35±0.40** | 3.02±0.51** | 3.39±0.34*** | 14.64±2.17*** | 30.20±5.59*** |
| MAGLZ-II-18 a High-dose group | 10 | 1.96±0.46*** | 2.60±0.58*** | 3.00±0.54*** | 13.90±1.13*** | 29.44±4.37*** |

Compared with the blank group, $^{\#P<}$0.05, $^{\#\#}$P< 0.01, $^{\#\#\#}$P< 0.001; and compared with the model group, *P<0.05, **P<0.01 and ***P<0.001.

It can be seen from the experimental results shown in Table 10 that compared with the model group, the MAGLZ-II-11, MAGLZ-II-11a, MAGLZ-II-18a, MAGLZ-II-18 and MAGLZ-II-18c of the present invention may significantly decrease the AWR scoring, and the 5-HT level in colon and serum.

**Example 44 Influences of the MAGL inhibitor of the present invention on reserpine-induced mice migraine**

**1. Animal grouping and administration**

210 male ICR mice (20±2) g were divided into 21 groups randomly according to body weight, 10 pieces for each group. The specific grouping conditions are as follows: blank group: an equal volume of 0.5% sodium carboxymethylcellulose, ig

model group: an equal volume of 0.5% sodium carboxymethylcellulose, ig

positive control group: 0.5 mg/kg Zolmitriptan, ig

MAGLZ-II-11 low-dose group: 7.5 mg/kg, ig

MAGLZ-II-11 middle-dose group: 15 mg/kg, ig

MAGLZ-II-11 high-dose group: 30 mg/kg, ig

MAGLZ-II-11a low-dose group: 7.5 mg/kg, ig

MAGLZ-II-11a middle-dose group: 15 mg/kg, ig

MAGLZ-II-11a high-dose group: 30 mg/kg, ig

MAGLZ-II-18a low-dose group: 7.5 mg/kg, ig

MAGLZ-II-18a middle-dose group: 15 mg/kg, ig

MAGLZ-II-18a high-dose group: 30 mg/kg, ig

MAGLZ-II-18 low-dose group: 7.5 mg/kg, ig

MAGLZ-II-18 middle-dose group: 15 mg/kg, ig

MAGLZ-II-18 high-dose group: 30 mg/kg, ig

MAGLZ-II-18c low-dose group: 7.5 mg/kg, ig

MAGLZ-II-18c middle-dose group: 15 mg/kg, ig

MAGLZ-II-18c high-dose group: 30 mg/kg, ig

MAGLZ-II-10 low-dose group: 7.5 mg/kg, ig

MAGLZ-II-10 middle-dose group: 15 mg/kg, ig

MAGLZ-II-10 high-dose group: 30 mg/kg, ig

Each compound was prepared into a suspension by 0.5% sodium carboxymethylcellulose respectively, and then set according to the dose, and each administration group was administered intragastrically; the model group was administered intragastrically an equal volume of 0.5% sodium carboxymethylcellulose.

**2. Experimental method and data processing**

The blank control group was subcutaneously injected with normal saline, and the rest groups of mice were subcutaneously injected with a reserpine solution (0.2 mg/kg), 10 d in total; if the mice showed eye closure, squat and reduced activity, diarrhea, less food-intake, back arching and other typical reserpine symptoms after being injected with reserpine, the modeling was believed successful.

The mice in the blank control group and model group were administered intragastrically via a solvent from the 5th day after modeling, and the rest groups of mice were respectively administered corresponding medicaments and doses for intragastric administration for 10 consecutive days; 1 h later after the final administration, the mice were weighed; blood was taken at inner canthus to detect blood coagulation time; whole blood was taken, and cerebrum was taken on an ice and preserved at -20°C for the subsequent experiment.

End point of the experiment: model administration was continued for 10 d, detection and blood taking were performed 1 h later after the final administration on the 10th day, thus finishing the experiment.

Test indicator: the mice were weighed once every two days after the beginning of the experiment.

Behavioral test: tail suspension activity was performed; the position 1 cm away from the tail of the mice was glued by an adhesive tape on the 9th day of the test, and the adhesive tape was penetrated by a steel needle, and hung in a dark box such that the mice were hung upside down; then the mice struggling times (total number of overturning from hanging) within 2 min were observed.

The mice were weighed 1 h later after the final administration; blood of inner canthus was taken via a capillary to detect the blood coagulation time.

Blood was taken to detect the content of 5-HT in serum via high-performance liquid chromatography. Cerebrum was taken on an ice and precooled and cleaned slightly with 0.5% sodium carboxymethylcellulose, and then sucked dry with a filter paper, and placed into a cryogenic tube, and preserved at -20°C for further use, then the content of 5-HT in brain tissues was detected by high performance liquid chromatography.

5-HT test method: a sample was precipitated with 10% perchloric acid solution according to 1:1, and then centrifuged to take supernatant for test. Chromatographic conditions: the chromatographic column is a SHIMADZU VP-ODS $C_{18}$ column (250*4.6 mm, 5 um), the mobile phase is a methanol-0.01 mol/L potassium acetate buffer solution (10:90, V/V, PH is adjusted to 4.00 with 0.2 mol/L citric acid) for gradient elution; the column temperature is 25 °C, flow rate is 1.00 ml/min and test wavelength is 275 nm.

Data is presented by a mean±SD, and the difference among groups is subjected to statistics with t tests or a one-way ANOVA method, and P<0.05 shows a significant difference.

**3. Experimental results**

Experimental results are shown in Tables 11-13.

Table 11 Influences of the MAGL inhibitor of the present invention on blood coagulation time

| Grouping | Dose | Blood coagulation time (S) |
|---|---|---|
| Normal group | - | 352.50±37.48 |
| Model group | - | 115.38±24.05*** |
| Zolmitriptan | 0.5 mg/kg | 185.12±38.86### |
| MAGLZ-II-18a low-dose group | 7.5 mg/kg | 137.25±67.22 |
| MAGLZ-II-18a middle-dose group | 15 mg/kg | 191.50±63.66### |
| MAGLZ-II-18a high-dose group | 30 mg/kg | 252.25±30.99### |
| MAGLZ-II-11 low-dose group | 7.5 mg/kg | 222.5±35.52### |
| MAGLZ-II-11 middle-dose group | 15 mg/kg | 326.88±45.9### |
| MAGLZ-II -11 high-dose group | 30 mg/kg | 336.88±33.09### |
| MAGLZ-II-10 low-dose group | 7.5 mg/kg | 113.25±23.31 |
| MAGLZ-II-10 middle-dose group | 15 mg/kg | 125.25±19.31 |
| MAGLZ-II -10 high-dose group | 30 mg/kg | 148.00±30.23# |
| MAGLZ-II-11a low-dose group | 7.5 mg/kg | 136.75±33.22 |
| MAGLZ-II-11a middle-dose group | 15 mg/kg | 256.88±42.93### |
| MAGLZ-II-11a high-dose group | 30 mg/kg | 300.63±45.26### |
| MAGLZ-II-18c low-dose group | 7.5 mg/kg | 148.00±30.23# |
| MAGLZ-II-18c middle-dose group | 15 mg/kg | 198.63±21.36### |
| MAGLZ-II -18c high-dose group | 30 mg/kg | 237.13±28.75### |
| MAGLZ-II-18 low-dose group | 7.5 mg/kg | 186.5±51.71### |
| MAGLZ-II-18 middle-dose group | 15 mg/kg | 212.63±46.02### |
| MAGLZ-II -18 high-dose group | 30 mg/kg | 241.5±41.63### |

Compared with the model group, #P<0.05, ##P<0.01, ###P<0.001.
It can be seen from the experimental results shown in Table 11 that compared with the model group, the MAGLZ-II-11, MAGLZ-II-11a, MAGLZ-II-18a, MAGLZ-II-18 and MAGLZ-II-18c of the present invention may significantly extend the blood coagulation time.

Table 12 Influences of the MAGL inhibitor of the present invention on the times of tail suspension

| Grouping | Dose | Times |
|---|---|---|
| Normal group | - | 22.38±3.38 |
| Model group | - | 8.38±2.62*** |
| Zolmitriptan | 0.5 mg/kg | 18.13+3.94### |
| MAGLZ-II-18a low-dose group | 7.5 mg/kg | 11.38±2.67# |
| MAGLZ-II-18a middle-dose group | 15 mg/kg | 13.38±2.67### |
| MAGLZ-II-18a high-dose group | 30 mg/kg | 18.50±2.00### |
| MAGLZ-II-10 low-dose group | 7.5 mg/kg | 9.25±2.66 |
| MAGLZ-II -10 middle-dose group | 15 mg/kg | 10.63±1.19# |
| MAGLZ-II -10 high-dose group | 30 mg/kg | 11.63±1.85# |
| MAGLZ-II-11 low-dose group | 7.5 mg/kg | 14.13±1.89### |
| MAGLZ-II -11 middle-dose group | 15 mg/kg | 16.13±1.73### |
| MAGLZ-II -11 high-dose group | 30 mg/kg | 23.00±3.59### |
| MAGLZ-II-11a low-dose group | 7.5 mg/kg | 16.13±3.68### |
| MAGLZ-II-11a middle-dose group | 15 mg/kg | 19.88±4.09### |
| MAGLZ-II-11a high-dose group | 30 mg/kg | 20.75±4.98### |

(continued)

| Grouping | Dose | Times |
|---|---|---|
| MAGLZ-II-18c low-dose group | 7.5 mg/kg | 10.13±2.3### |
| MAGLZ-II -18c middle-dose group | 15 mg/kg | 12.75±2.6### |
| MAGLZ-II -18c high-dose group | 30 mg/kg | 15.88±3.8### |
| MAGLZ-II-18 low-dose group | 7.5 mg/kg | 11.88±4.02### |
| MAGLZ-II -18 middle-dose group | 15 mg/kg | 13.75±4.56### |
| MAGLZ-II -18 high-dose group | 30 mg/kg | 16.51±4.38### |

Compared with the model group, #P<0.05, ##P<0.01, ###P<0.001.

It can be seen from the experimental results shown in Table 12 that compared with the model group, the MAGLZ-II-11, MAGLZ-II-11a, MAGLZ-II-18a, MAGLZ-II-18 and MAGLZ-II-18c of the present invention may significantly increase the times of the tail suspension activity.

Table 13 Influences of the MAGL inhibitor of the present invention on a brain tissue 5-hydroxytryptamine

| Grouping | Dose | 5-HT (nmol/g) |
|---|---|---|
| Normal group | - | 3.36±0.4 |
| Model group | - | 0.88±0.23*** |
| Zolmitriptan | 0.5 mg/kg | 1.93±0.37### |
| MAGLZ-II-18a low-dose group | 7.5 mg/kg | 1.21±0.17### |
| MAGLZ-II-18a middle-dose group | 15 mg/kg | 1.65±0.18### |
| MAGLZ-II-18a high-dose group | 30 mg/kg | 2.76±0.34### |
| MAGLZ-II-10 low-dose group | 7.5 mg/kg | 0.85±0.20 |
| MAGLZ-II -10 middle-dose group | 15 mg/kg | 1.05±0.14 |
| MAGLZ-II -10 high-dose group | 30 mg/kg | 0.90±0.21# |
| MAGLZ-II-11 low-dose group | 7.5 mg/kg | 1.54±0.27### |
| MAGLZ-II -11 middle-dose group | 15 mg/kg | 2.10±0.22### |
| MAGLZ-II -11 high-dose group | 30 mg/kg | 3.63±5.34### |
| MAGLZ-II-11a low-dose group | 7.5 mg/kg | 1.35±0.38## |
| MAGLZ-II-11a middle-dose group | 15 mg/kg | 1.86±0.29### |
| MAGLZ-II-11a high-dose group | 30 mg/kg | 2.97±0.28### |
| MAGLZ-II-18c low-dose group | 7.5 mg/kg | 1.14±0.22# |
| MAGLZ-II -18c middle-dose group | 15 mg/kg | 1.37±0.34## |
| MAGLZ-II -18c high-dose group | 30 mg/kg | 1.79±0.32### |
| MAGLZ-II-18 low-dose group | 7.5 mg/kg | 1.13±0.45# |
| MAGLZ-II -18 middle-dose group | 15 mg/kg | 1.54±0.54### |
| MAGLZ-II -18 high-dose group | 30 mg/kg | 2.08±0.59### |

Compared with the model group, #P<0.05, ##P<0.01, ###P<0.001.

It can be seen from the experimental results shown in Table 13 that compared with the model group, the MAGLZ-II-11, MAGLZ-II-11a, MAGLZ-II-18a, MAGLZ-II-18 and MAGLZ-II-18c of the present invention may significantly increase the level of 5-HT in the brain tissue of reserpine-induced low 5-HT model mice.

**Example 45 Study on the effect of the** MAGL **inhibitor on mice ulcerative colitis**

**1. Animal grouping and administration**

Healthy male SPF-level BALB/c mice (8-week old and body mass: (20±2) g).

The mice were quarantined for 7 d after entering to a room, and healthy male mice were selected as subject animals. Major examination contents within the quarantine period include but not limited to: whether to be consistent with the quantity and body indicators of the animals required during application, general state, and body weight; animals not

passing through the above quarantine items were not brought into the test.

Modeling: the mice were randomly divided into 21 groups at the end of the quarantine, 10 pieces for each group. The mice in the normal group drank normal water freely, and the rest groups of mice drank 1.0%-1.5% DSS solution freely for modeling, 7 d later after modeling, the mice in the modeling group drank normal water freely for 7 d, and 14 d served as a modeling period for four continuous periods, and the mice in the modeling group were administered at the same time.

Blank group: an equal volume of 0.5% sodium carboxymethylcellulose, ig

Model group: an equal volume of 0.5% sodium carboxymethylcellulose, ig

Positive control group: Mesalazine (100 mg/kg), ig

MAGLZ-II-11 low-dose group: 7.5 mg/kg, ig

MAGLZ-II-11 middle-dose group: 15 mg/kg, ig

MAGLZ-II-11 high-dose group: 30 mg/kg, ig

MAGLZ-II-11a low-dose group: 7.5 mg/kg, ig

MAGLZ-II-11a middle-dose group: 15 mg/kg, ig

MAGLZ-II-11a high-dose group: 30 mg/kg, ig

MAGLZ-II-18a low-dose group: 7.5 mg/kg, ig

MAGLZ-II-18a middle-dose group: 15 mg/kg, ig

MAGLZ-II-18a high-dose group: 30 mg/kg, ig

MAGLZ-II-18 low-dose group: 7.5 mg/kg, ig

MAGLZ-II-18 middle-dose group: 15 mg/kg, ig

MAGLZ-II-18 high-dose group: 30 mg/kg, ig

MAGLZ-II-18c low-dose group: 7.5 mg/kg, ig

MAGLZ-II-18c middle-dose group: 15 mg/kg, ig

MAGLZ-II-18c high-dose group: 30 mg/kg, ig

MAGLZ-II-10 low-dose group: 7.5 mg/kg, ig

MAGLZ-II-10 middle-dose group: 15 mg/kg, ig

MAGLZ-II-10 high-dose group: 30 mg/kg, ig

Each compound was prepared into a suspension by 0.5% sodium carboxymethylcellulose respectively, and then set according to the dose, and each administration group was administered intragastrically; the model group was administered intragastrically an equal volume of 0.5% sodium carboxymethylcellulose.

**2. Test process and method**

End point of the experiment: experimental administration was performed for four periods, and dissection was performed on the 3rd and 4th day of the recovery period of the 4th period.

Test indicators: the mice were weighed twice a week after the beginning of the administration; diarrhea and hematochezia of the mice were observed, and times of hematochezia were recorded.

At the end of the experiment, the mice were dissected and weighed, and narcotized to take blood, then serum was preserved at -80°C. Spleen was taken and weighed to calculate the spleen index. The content of IL-1β in serum was detected via ELISA. Colorectum was cut and taken to measure the length of colorectum, then immobilized by formaldehyde and dyed by HE to detect the pathological change.
Data is presented by a mean±SD, and the difference among groups is subjected to statistics with t tests or a one-way ANOVA method, and $P<0.05$ shows a significant difference.

**3. Result and discussion**

Experimental results are shown in Table 14.

Table 14 Influences of the MAGL inhibitor of the present invention on the colon length, hematochezia times and IL-1 content of ulcerative colitis mice

| Group | Quantity | Colon length (cm) | Total times of IL-1 content hematochezia within (pg/mL) the four periods | |
|---|---|---|---|---|
| Blank group | 10 | 8.22±1.45 | 0±0 | 35.36±5.92 |
| Model group | 10 | 5.70±0.78$^{\#\#\#}$ | 38.60±2.80$^{\#\#\#}$ | 55.53±3.62$^{\#\#\#}$ |
| Mesalazine (100 mg/kg) | 10 | 6.66±0.68** | 32.80±4.96** | 48.33±6.49** |
| MAGLZ-II-11 low-dose group | 10 | 6.88±0.89** | 32.00±4.94** | 44.57±10.32** |
| MAGLZ-II-11 middle-dose group | 10 | 7.26±0.67*** | 27.20±5.39*** | 41.40±9.50*** |
| MAGLZ-II-11 high-dose group | 10 | 7.68±0.75*** | 24.80±5.43*** | 33.83±7.14*** |
| MAGLZ-II-18a low-dose group | 10 | 6.51±0.90* | 33.60±5.83** | 47.32±9.06* |
| MAGLZ-II-18a middle-dose group | 10 | 7.06±1.20** | 29.90±8.95** | 43.77±10.69** |
| MAGLZ-II-18a high-dose group | 10 | 7.72±0.92*** | 27.90±7.28*** | 36.22±4.31*** |
| MAGLZ-II-18c low-dose group | 10 | 6.25±1.17 | 43.10±7.59 | 48.08±13.18 |
| MAGLZ-II-18c middle-dose group | 10 | 6.47±0.71* | 43.20±5.31* | 44.09±9.13** |
| MAGLZ-II-18c high-dose group | 10 | 6.65±0.67** | 31.80±6.6** | 45.10±8.88** |
| MAGLZ-II-11a low-dose group | 10 | 7.21±1.10** | 28.80±7.04** | 42.01±13.92** |
| MAGLZ-II-11a middle-dose group | 10 | 7.20±0.97** | 29.10±7.14** | 40.70±8.50*** |
| MAGLZ-II-11a high-dose group | 10 | 7.56±0.74*** | 27.90±3.73*** | 38.61±10.98*** |
| MAGLZ-II-10 low-dose group | 10 | 5.92±0.66 | 38.40±5.02 | 51.81±7.86 |
| MAGLZ-II-10 middle-dose group | 10 | 5.72±0.60 | 38.20±7.83 | 50.83±7.79 |
| MAGLZ-II-10 high-dose group | 10 | 6.35+0.61* | 35.15±4.52* | 47.25±6.37* |
| MAGLZ-II-18 low-dose group | 10 | 6.44±0.64* | 32.80±4.64** | 46.15±9.65* |
| MAGLZ-II-18 middle-dose group | 10 | 6.37+0.39* | 33.90±4.77* | 49.01±5.65* |
| MAGLZ-II-18 high-dose group | 10 | 6.73±0.63** | 32.80±4.37** | 46.98±7.56** |

Compared with the blank group, $^{\#P<}$0.05, $^{\#\#}$P<0.01, $^{\#\#\#}$P<0.001;
and compared with the model group, *P<0.05, **P<0.01 and ***P< 0.001.
Compared with the model group, the MAGLZ-II-11, MAGLZ-II-11a, MAGLZ-II-18a, MAGLZ-II-18 and MAGLZ-II-18c of the present invention may significantly increase the colon length, reduce the total times of hematochezia and decrease the IL-1 content.

## Claims

1. A compound of Formula I or a pharmaceutically acceptable salt thereof:

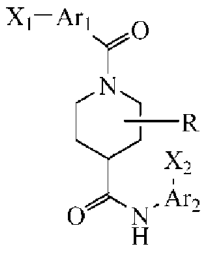

I ,

wherein R is H or $C_{1-5}$ alkyl, and the $C_{1-5}$ alkyl is selected from methyl, ethyl, propyl, isopropyl, n-butyl, isobutyl, neo-butyl, n-pentyl, isopentyl, neo-pentyl, cyclopentyl, or $C_{1-5}$ alkyl substituted by halogen, hydroxy, carboxyl, $C_{1-3}$ alkyl, $C_{1-3}$ alkoxy, $C_{3-8}$ cycloalkyl, $C_{3-8}$ heterocyclyl, $C_{3-8}$ cycloalkoxy, aryl, and heteroaryl; the number of R is 0, 1, 2, 3 or 4;
the $Ar_1$ is one or more of phenyl, naphthyl, phenoxy-phenyl and biphenyl;
the $X_1$ is hydroxy, at any substitution position;
the $Ar_2$ is phenyl; and
the $X_2$ is one or more of H, methyl, methoxy, hydroxy, F and Cl, at any substitution position.

**2.** A compound of Formula I or a pharmaceutically acceptable salt thereof:

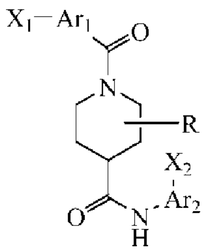

I ,

wherein R is H or $C_{1-5}$ alkyl, and the $C_{1-5}$ alkyl is selected from methyl, ethyl, propyl, isopropyl, n-butyl, isobutyl, neo-butyl, n-pentyl, isopentyl, neo-pentyl, cyclopentyl, or $C_{1-5}$ alkyl substituted by halogen, hydroxy, carboxyl, $C_{1-3}$ alkyl, $C_{1-3}$ alkoxy, $C_{3-8}$ cycloalkyl, $C_{3-8}$ heterocyclyl, $C_{3-8}$ cycloalkoxy, aryl, and heteroaryl; the number of R is 0, 1, 2, 3 or 4;
the $Ar_1$ is one or more of benzofuryl, benzimidazolyl, benzothiophenyl, quinolyl, isoquinolyl and purinyl, at any substitution position;
the $X_1$ is one or more of H, methyl, methoxy, hydroxy, F, Cl and Br, at any substitution position;
the $Ar_2$ is phenyl;
the $X_2$ is one or more of H, methyl, methoxy, hydroxy, F and Cl, at any substitution position.

**3.** The compound or a pharmaceutically acceptable salt thereof according to claim 1, wherein the compound is selected from the group consisting of:

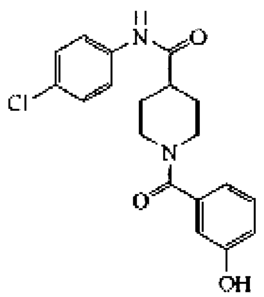

MAGLZ-II-08

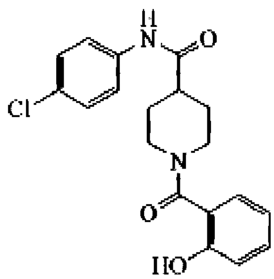

MAGLZ-II-09

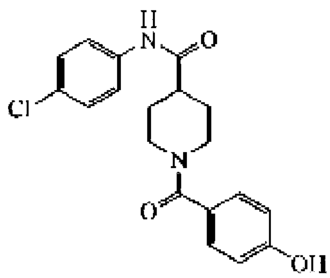

MAGLZ-II-10

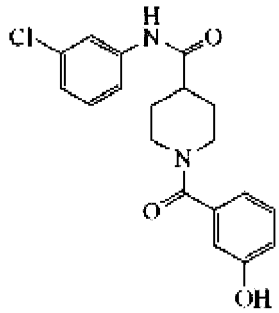
MAGLZ-II-11

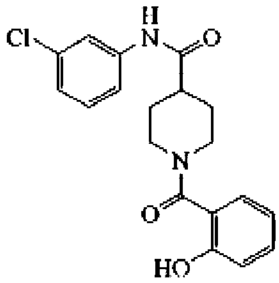
MAGLZ-II-12

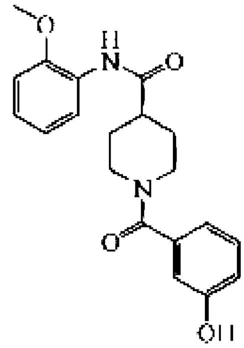
MAGLZ-II-13

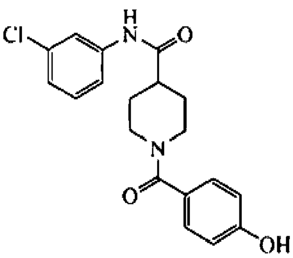
MAGLZ-II-20

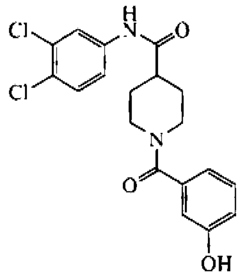
MAGLZ-II-22

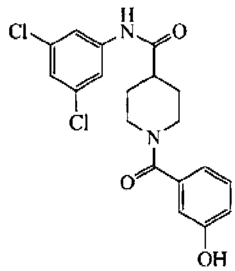
MAGLZ-II-23

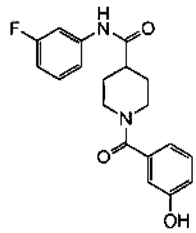
MAGLZ-II-26

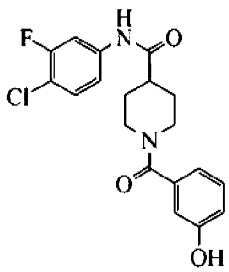
MAGLZ-II-27 and

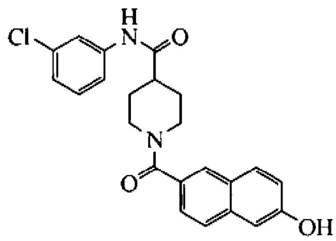
MAGLZ-II-29 .

**4.** The compound or a pharmaceutically acceptable salt thereof according to claim 2, wherein the compound is selected from the group consisting of:

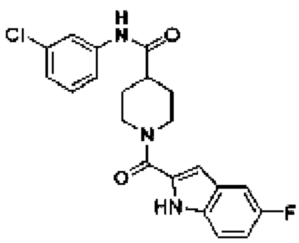
MAGLZ-II-18(a) and

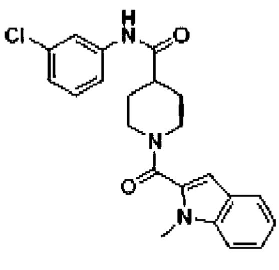
MAGLZ-II-18(c) .

**5.** A compound or a pharmaceutically acceptable salt thereof, wherein the compound is selected from the group consisting of:

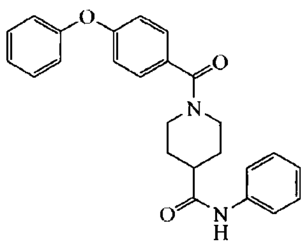

MAGLZ-II-01

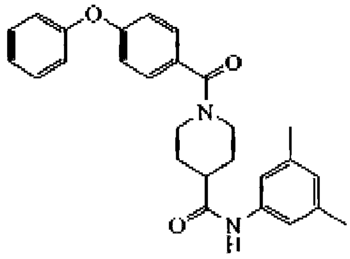

MAGLZ-II-02

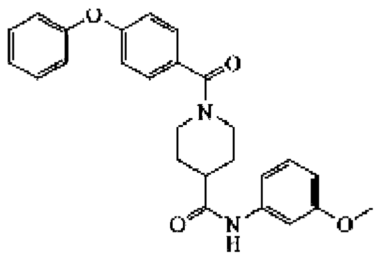

MAGLZ-II-03

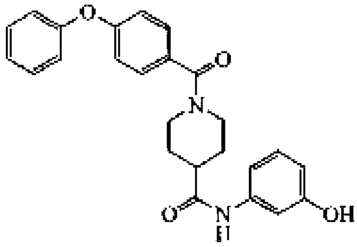

MAGLZ-II-04

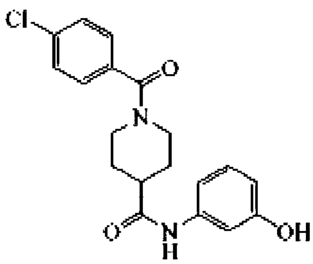

MAGLZ-II-06

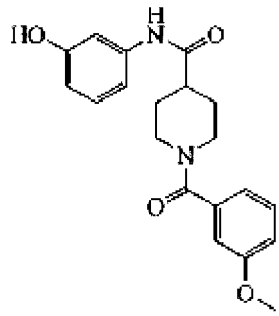

MAGLZ-II-15

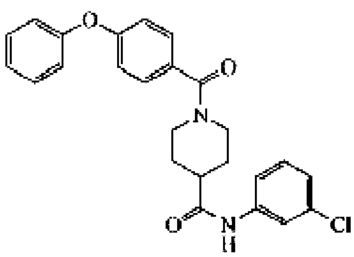

MAGLZ-II-16

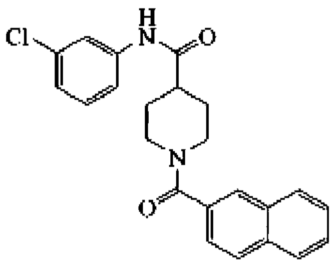

MAGLZ-II-17

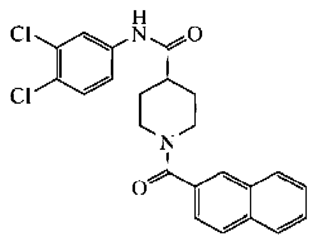

MAGLZ-II-24

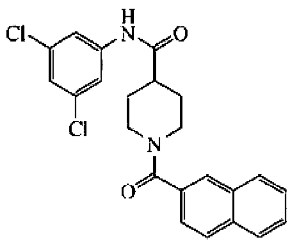

MAGLZ-II-25

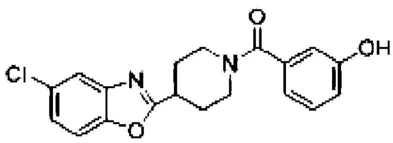

MAGLZ-II-30

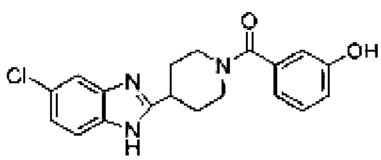

MAGLZ-II-31

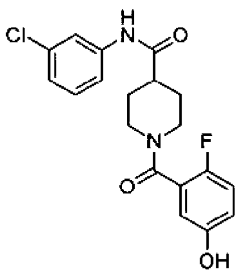

MAGLZ-II-11(a)

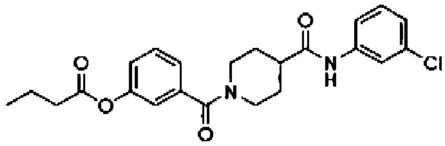

MAGLZ-II-11F

and

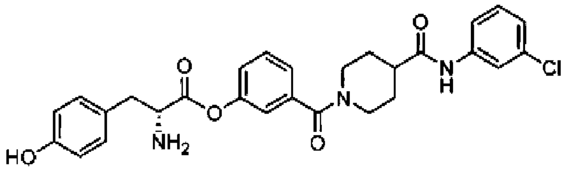

MAGLZ-II-11J

.

**6.** A method of synthesizing the compound of Formula I according to claim 1, comprising

the following specific steps:

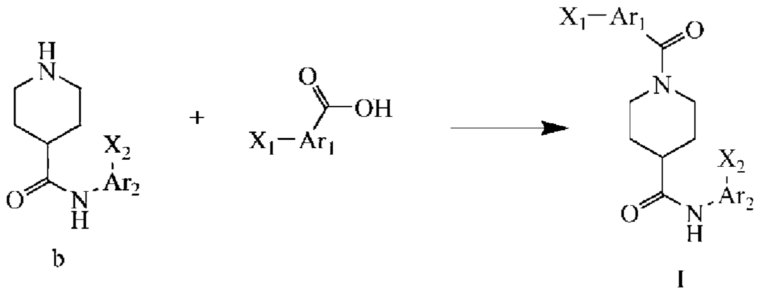

b

I

, ,

wherein b or a salt of b is reacted with

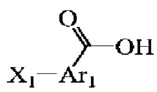

to obtain I; the $X_1$, the $X_2$, the $Ar_1$ and the $Ar_2$ are as described in claim 1,
preferably, wherein the b or the salt of b is reacted with

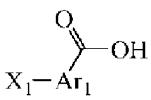

to obtain I; a condensating agent and an alkali are added in this step to facilitate the reaction; wherein, the condensating agent is selected from HBTU, DMC, HOBT, HOBT/EDCI, HATU, HATU/DIEPA, DCC, CDI, and isopropyl chloroformate,
preferably, wherein the b or the salt of b is obtained by deprotection of a;

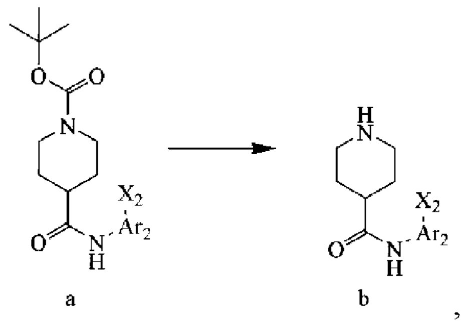

preferably, wherein the a or the salt of a is obtained by reacting sm with

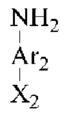

or a salt thereof;

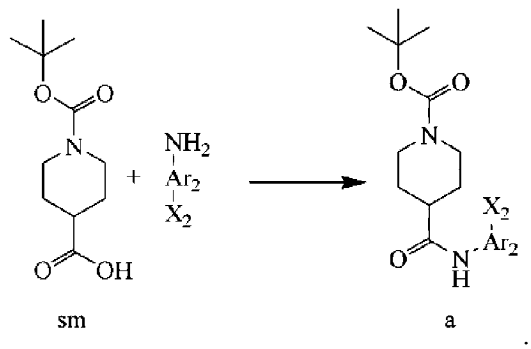

7. A pharmaceutical composition, comprising the compound or a pharmaceutically acceptable salt thereof of any one of claims 1-5, and a pharmaceutically acceptable carrier.

8. The compound or a pharmaceutically acceptable salt thereof of any one of claims 1-5, for use in treating a MAGL-mediated disease or condition,
wherein the disease or condition is selected from: metabolic disorders, nephrosis, emesis or vomiting or nausea, eating disorders, neuropathy, schizophrenia, depressive disorders, bipolar disorders, fremitus, dyskinesia, abstinence syndromes, traumatic brain injury, non-traumatic brain injury, spinal cord injury, epileptic seizure, conditions associated with abnormal cell growth or proliferation, inflammatory conditions, immune system conditions, irritable bowel syndromes, ulcer colonitis, acute stress disorders, substance-inducing anxiety, obsessive-compulsive disorders, anxiety disorders; attention deficiency disorders, attention deficit hyperactivity disorders, pains, migraine, demyelinating diseases, and cognitive impairments.

9. The compound or a pharmaceutically acceptable salt thereof for use of claim 8, wherein the conditions associated with abnormal cell growth or proliferation is benign tumors or cancers.

10. A method for inhibiting MAGL *in-vitro,* comprising contacting the MAGL with the compound or a pharmaceutically acceptable salt thereof of any one of claims 1-5.

11. A compound or a pharmaceutically acceptable salt thereof for use in treating a MAGL-mediated disease or condition, wherein the compound is selected from the group consisting of:

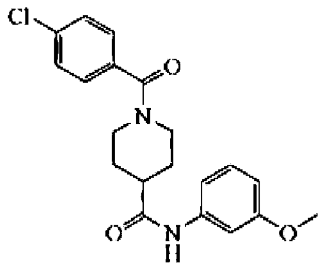

MAGLZ-II-05

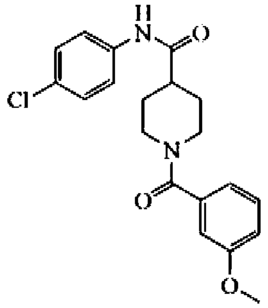

MAGLZ-II-07

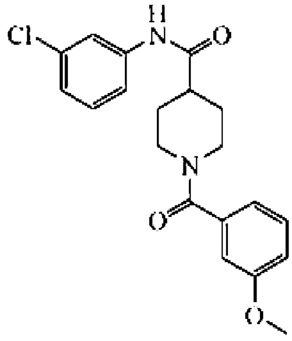

MAGLZ-II-14

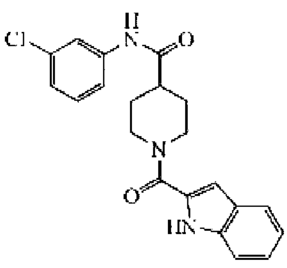

MAGLZ-II-18

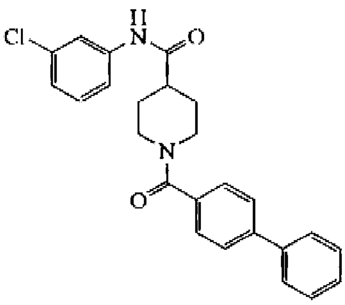

MAGLZ-II-19

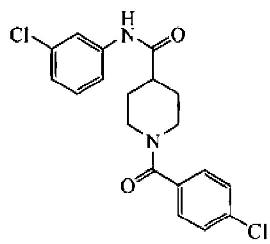

MAGLZ-II-21

,

and the MAGL-mediated disease or condition is selected from the group consisting of: metabolic disorders, nephrosis, emesis or vomiting or nausea, eating disorders, neuropathy, schizophrenia, depressive disorders, bipolar disorders, fremitus, dyskinesia, abstinence syndromes, traumatic brain injury, non-traumatic brain injury, spinal cord injury, epileptic seizure, conditions associated with abnormal cell growth or proliferation [such as, benign tumors or cancers], inflammatory conditions, immune system conditions, irritable bowel syndromes, ulcer colonitis, acute stress disorders, substance-inducing anxiety, obsessive-compulsive disorders, anxiety disorders; attention deficiency disorders, attention deficit hyperactivity disorders, pains, migraine, demyelinating diseases, and cognitive impairments.

**Patentansprüche**

**1.** Verbindung von Formel I oder ein pharmazeutisch annehmbares Salz davon:

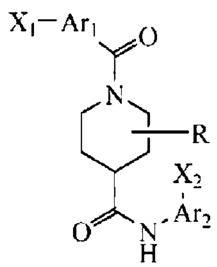

I ,

wobei R H oder $C_{1-5}$-Alkyl ist und das $C_{1-5}$-Alkyl ausgewählt ist aus Methyl, Ethyl, Propyl, Isopropyl, n-Butyl, Isobutyl, Neobutyl, n-Pentyl, Isopentyl, Neopentyl, Cyclopentyl oder $C_{1-5}$-Alkyl, substituiert durch Halogen, Hydroxy, Carboxyl, $C_{1-3}$-Alkyl, $C_{1-3}$-Alkoxy, $C_{3-8}$-Cycloalkyl, $C_{3-8}$-Heterocyclyl, $C_{3-8}$-Cycloalkoxy, Aryl und Heteroaryl; die Anzahl von R 0, 1, 2, 3 oder 4 beträgt;

$Ar_1$ eines oder mehrere von Phenyl, Naphthyl, Phenoxy-phenyl und Biphenyl ist;
$X_1$ Hydroxy an jeder beliebigen Substitutionsposition ist;

$Ar_2$ Phenyl ist; und
$X_2$ eines oder mehrere von H, Methyl, Methoxy, Hydroxy, F und Cl an jeder beliebigen Substitutionsposition ist.

**2.** Verbindung von Formel I oder ein pharmazeutisch annehmbares Salz davon:

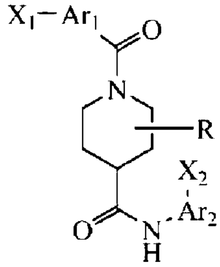

I ,

wobei R H oder $C_{1-5}$-Alkyl ist und das $C_{1-5}$-Alkyl ausgewählt ist aus Methyl, Ethyl, Propyl, Isopropyl, n-Butyl, Isobutyl, Neobutyl, n-Pentyl, Isopentyl, Neopentyl, Cyclopentyl oder $C_{1-5}$-Alkyl, substituiert durch Halogen, Hydroxy, Carboxyl, $C_{1-3}$-Alkyl, $C_{1-3}$-Alkoxy, $C_{3-8}$-Cycloalkyl, $C_{3-8}$-Heterocyclyl, $C_{3-8}$-Cycloalkoxy, Aryl und Heteroaryl; die Anzahl von R 0, 1, 2, 3 oder 4 beträgt;
$Ar_1$ eines oder mehrere von Benzofuryl, Benzimidazolyl, Benzothiophenyl, Chinolyl, Isochinolyl und Purinyl an jeder beliebigen Substitutionsposition ist;
$X_1$ eines oder mehrere von H, Methyl, Methoxy, Hydroxy, F, Cl und Br an jeder beliebigen Substitutionsposition ist;
$Ar_2$ Phenyl ist;
$X_2$ eines oder mehrere von H, Methyl, Methoxy, Hydroxy, F oder Cl an jeder beliebigen Substitutionsposition ist.

**3.** Verbindung oder ein pharmazeutisch annehmbares Salz davon nach Anspruch 1, wobei die Verbindung ausgewählt ist aus der Gruppe bestehend aus:

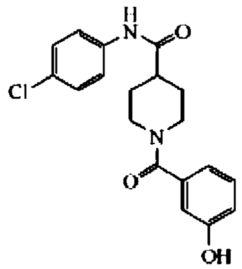

MAGLZ-II-08

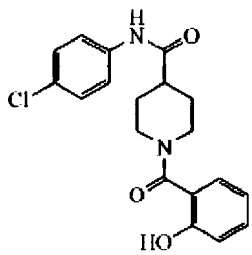

MAGLZ-II-09

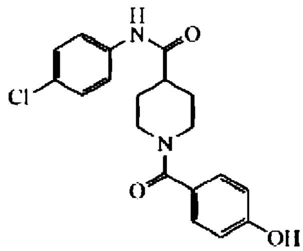

MAGLZ-II-10

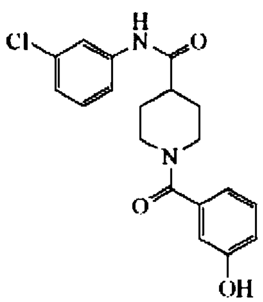

MAGLZ-II-11

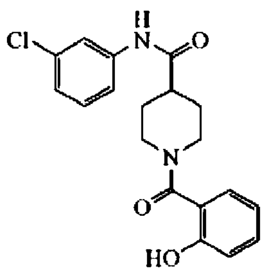

MAGLZ-II-12

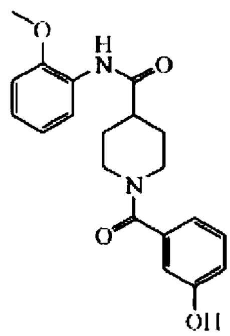

MAGLZ-II-13

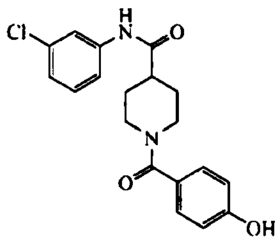

MAGLZ-II-20

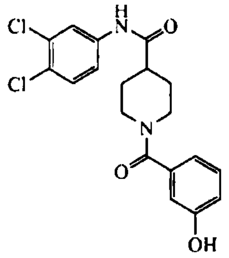

MAGLZ-II-22

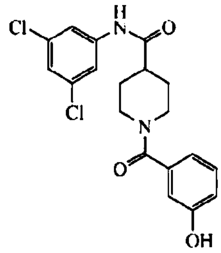

MAGLZ-II-23

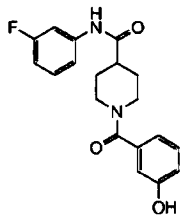

MAGLZ-II-26

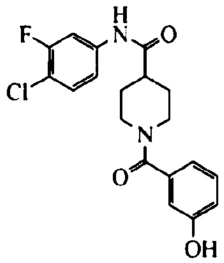

MAGLZ-II-27

und

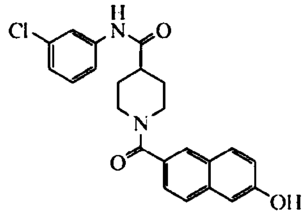

MAGLZ-II-29

.

4. Verbindung oder ein pharmazeutisch annehmbares Salz davon nach Anspruch 2, wobei die Verbindung ausgewählt ist aus der Gruppe bestehend aus:

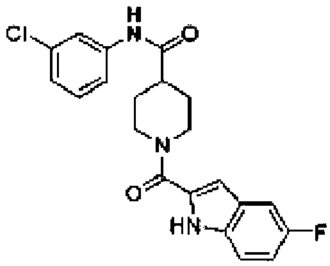

MAGLZ-II-18(a)

und

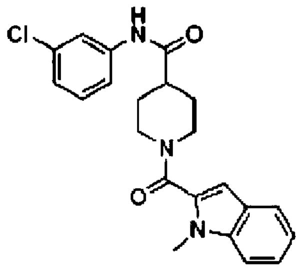

MAGLZ-II-18(c)

.

5. Verbindung oder ein pharmazeutisch annehmbares Salz davon, wobei die Verbindung ausgewählt ist aus der Gruppe bestehend aus:

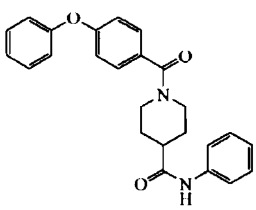

MAGLZ-II-01

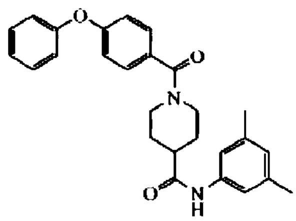

MAGLZ-II-02

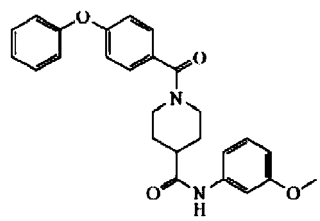

MAGLZ-II-03

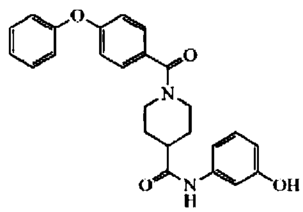

MAGLZ-II-04

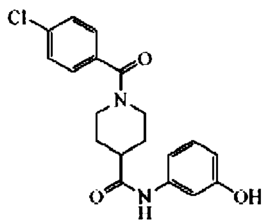

MAGLZ-II-06

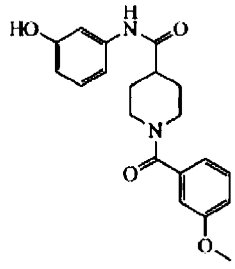

MAGLZ-II-15

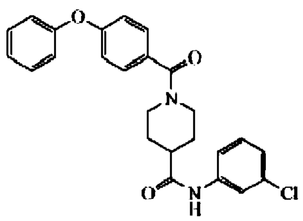

MAGLZ-II-16

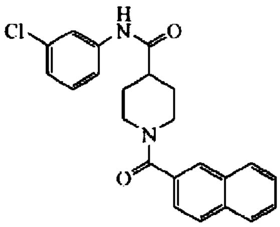

MAGLZ-II-17

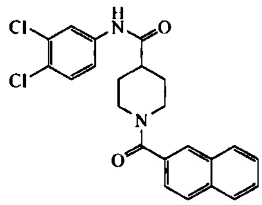

MAGLZ-II-24

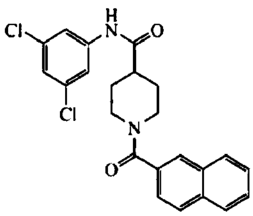

MAGLZ-II-25

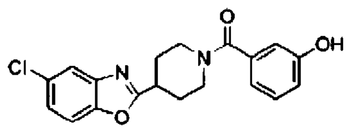

MAGLZ-II-30

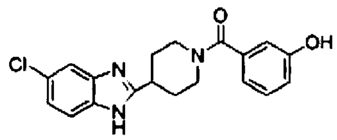

MAGLZ-II-31

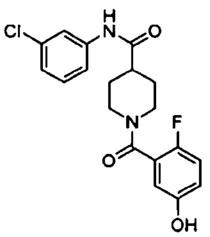

MAGLZ-II-11(a)

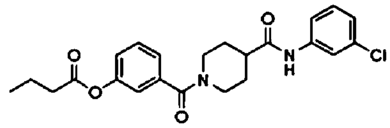

MAGLZ-II-11F

und

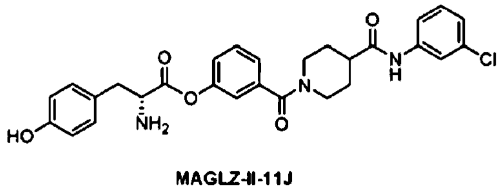

MAGLZ-II-11J

.

**6.** Verfahren zur Synthese der Verbindung von Formel I nach Anspruch 1, das die folgenden spezifischen Schritte beinhaltet:

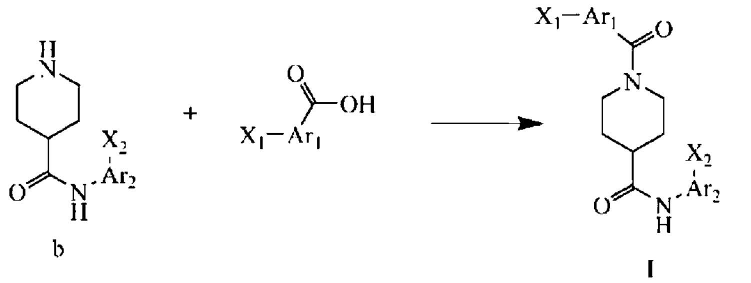

wobei b oder ein Salz von b mit

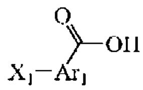

umgesetzt wird, um I zu erhalten; wobei $X_1$, $X_2$, $Ar_1$ und $Ar_2$ wie in Anspruch 1 beschrieben sind, wobei vorzugsweise b oder das Salz von b mit

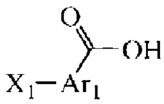

umgesetzt wird, um I zu erhalten; in diesem Schritt ein Kondensationsmittel und ein Alkali zugegeben werden, um die Reaktion zu erleichtern; wobei das Kondensationsmittel ausgewählt wird aus HBTU, DMC, HOBT, HOBT/EDCI, HATU, HATU/DIEPA, DCC, CDI und Isopropylchlorformiat,
wobei vorzugsweise das b oder das Salz von b durch Entschützen von a erhalten wird;

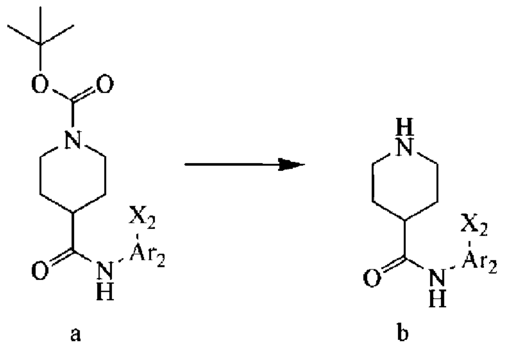

wobei vorzugsweise a oder das Salz von a durch Umsetzen von sm mit

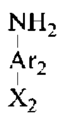

oder einem Salz davon erhalten wird;

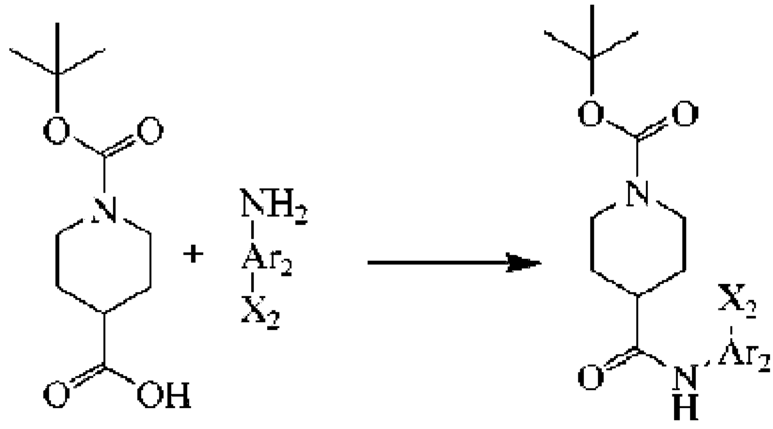

sm a

.

7. Pharmazeutische Zusammensetzung, die die Verbindung oder ein pharmazeutisch annehmbares Salz davon nach einem der Ansprüche 1-5 und einen pharmazeutisch annehmbaren Träger umfasst.

8. Verbindung oder ein pharmazeutisch annehmbares Salz davon nach einem der Ansprüche 1-5 zur Verwendung bei der Behandlung einer MAGL-vermittelten Erkrankung oder eines MAGL-vermittelten Zustands, wobei die Erkrankung oder der Zustand ausgewählt ist aus: Stoffwechselstörungen, Nephrose, Emesis oder Erbrechen oder Übelkeit, Essstörungen, Neuropathie, Schizophrenie, depressiven Störungen, bipolaren Störungen, Fremitus, Dyskinesie, Entzugssyndromen, traumatischen Hirnverletzungen, nicht-traumatischen Hirnverletzungen, Rückenmarksverletzungen, epileptischen Anfällen, Zuständen, die mit abnormalem/r Zellwachstum oder -proliferation assoziiert sind, entzündlichen Zuständen, Erkrankungen des Immunsystems, Reizdarmsyndromen, ulzerativer Kolitis, akuten Belastungsstörungen, substanzinduzierter Angst, Zwangsstörungen, Angststörungen; Aufmerksamkeitsdefizitstörungen, Aufmerksamkeitsdefizit-Hyperaktivitätsstörungen, Schmerzen, Migräne, demyelinisierenden Erkrankungen und kognitiven Beeinträchtigungen.

9. Verbindung oder ein pharmazeutisch annehmbares Salz davon zur Verwendung nach Anspruch 8, wobei die mit abnormalem/r Zellwachstum oder -proliferation assoziierten Erkrankungen gutartige Tumoren oder Krebserkrankungen sind.

10. Verfahren zur *In-vitro-Hemmung* von MAGL, das das Inkontaktbringen von MAGL mit der Verbindung oder einem pharmazeutisch annehmbaren Salz davon nach einem der Ansprüche 1 bis 5 beinhaltet.

11. Verbindung oder ein pharmazeutisch annehmbares Salz davon zur Verwendung bei der Behandlung einer/s MAGL-vermittelten Erkrankung oder Zustands, wobei die Verbindung ausgewählt ist aus der Gruppe bestehend aus:

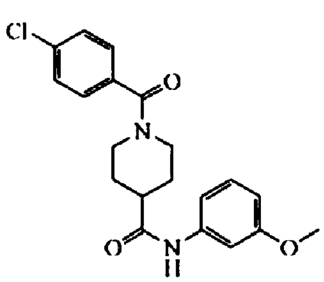

MAGLZ-II-05

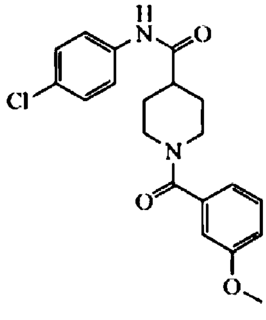

MAGLZ-II-07

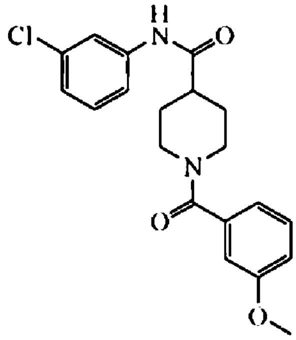

MAGLZ-II-14

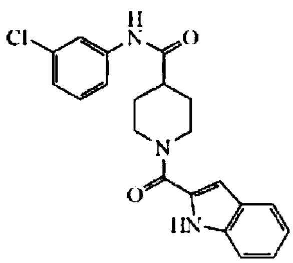

MAGLZ-II-18

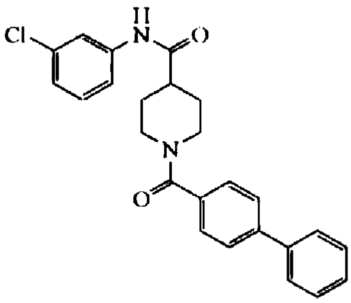

MAGLZ-II-19

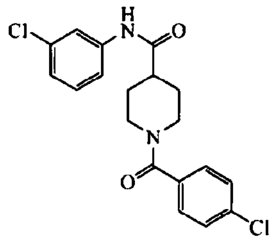

MAGLZ-II-21

,

und die/der MAGL-vermittelte Erkrankung oder Zustand ausgewählt ist aus der Gruppe bestehend aus: Stoffwechselstörungen, Nephrose, Emesis oder Erbrechen oder Übelkeit, Essstörungen, Neuropathie, Schizophrenie, depressiven Störungen, bipolaren Störungen, Fremitus, Dyskinesie, Entzugssyndromen, traumatischen Hirnverletzungen, nicht-traumatischen Hirnverletzungen, Rückenmarksverletzungen, epileptischen Anfällen, Zuständen im Zusammenhang mit abnormalem/r Zellwachstum oder -proliferation [wie beispielsweise gutartige Tumoren oder Krebserkrankungen], entzündlichen Erkrankungen, Erkrankungen des Immunsystems, Reizdarmsyndromen, ulzerativer Kolitis, akuten Belastungsstörungen, substanzinduzierten Angstzuständen, Zwangsstörungen, Angststörungen; Aufmerksamkeitsdefizitstörungen, Aufmerksamkeitsdefizit-Hyperaktivitätsstörungen, Schmerzen, Migräne, demyelinisierenden Erkrankungen und kognitiven Beeinträchtigungen.

## Revendications

**1.** Composé de la Formule 1 ou un sel pharmaceutiquement acceptable de celui-ci :

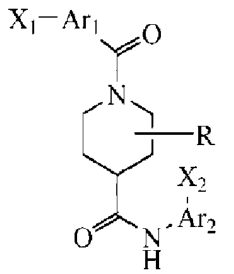

I

dans lequel R est H ou un alkyle en $C_{1-5}$, et l'alkyle en $C_{1-5}$ est sélectionné parmi méthyle, éthyle, propyle, isopropyle, n-butyle, isobutyle, néo-butyle, n-pentyle, isopentyle, néo-pentyle, cyclopentyle, ou l'alkyle en $C_{1-5}$ est substitué par halogène, hydroxy, carboxyle, alkyle en $C_{1-3}$, alcoxy en $C_{1-3}$, cycloalkyle en $C_{3-8}$, hétérocyclyle en $C_{3-8}$, cycloalcoxy en $C_{3-8}$, aryle et hétéroaryle ; le nombre de R est 0, 1, 2, 3, ou 4;
le $Ar_1$ est un ou plusieurs d'entre phényle, naphtyle, phénoxy-phényle et biphényle;
le $X_1$ est un hydroxy, à n'importe quelle position de substitution;
le $Ar_2$ est un phényle; et
le $X_2$ est un ou plusieurs d'entre H, méthyle, méthoxy, hydroxy, F et Cl, à n'importe quelle position de substitution.

**2.** Composé de la Formule 1 ou un sel pharmaceutiquement acceptable de celui-ci :

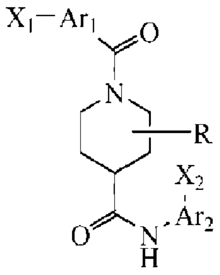

I

dans lequel R est H ou alkyle en en $C_{1-5}$, et l'alkyle en $C_{1-5}$ est sélectionné parmi méthyle, éthyle, propyle, isopropyle, n-butyle, isobutyle, néo-butyle, n-pentyle, isopentyle, néo-pentyle, cyclopentyle, ou l'alkyle en $C_{1-5}$ est substitué par halogène, hydroxy, carboxyle, alkyle en $C_{1-3}$, alcoxy en $C_{1-3}$, cycloalkyle en $C_{3-8}$, hétérocyclyle en $C_{3-8}$, cycloalcoxy en $C_{3-8}$, aryle et hétéroaryle ; le nombre de R est 0, 1, 2, 3, ou 4;
le $Ar_1$ est un ou plusieurs d'entre benzofuryle, benzimidazolyle, benzothiophényle, quinolyle, isoquinolyle et purinyle, à n'importe quelle position de substitution;
le $X_1$ est un ou plusieurs d'entre H, méthyle, méthoxy, hydroxy, F, Cl et Br, à n'importe quelle position de substitution;
le $Ar_2$ est un phényle;
le $X_2$ est un ou plusieurs d'entre H, méthyle, méthoxy, hydroxy, F et Cl, à n'importe quelle position de substitution.

**3.** Composé ou sel pharmaceutiquement acceptable de celui-ci selon la revendication 1, où le composé est sélectionné parmi le groupe composé de :

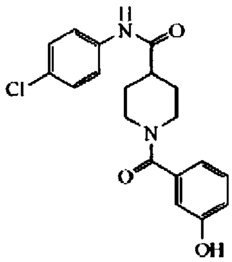

MAGLZ-II-08

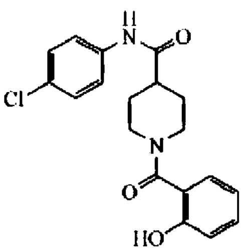

MAGLZ-II-09

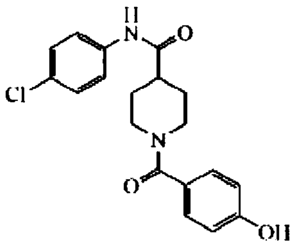

MAGLZ-II-10

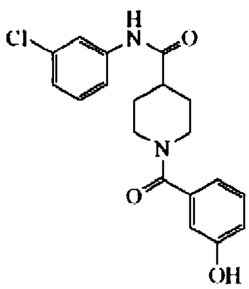

MAGLZ-II-11

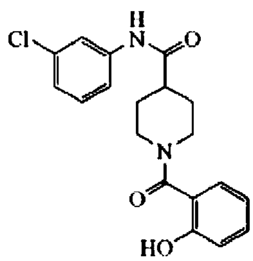

MAGLZ-II-12

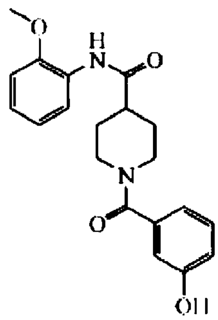

MAGLZ-II-13

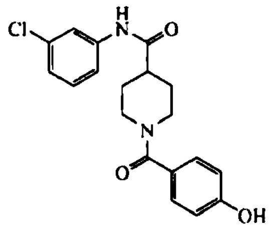

MAGLZ-II-20

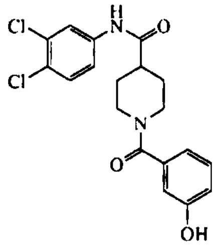

MAGLZ-II-22

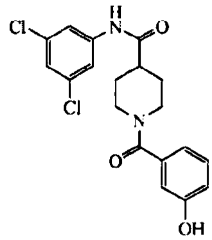

MAGLZ-II-23

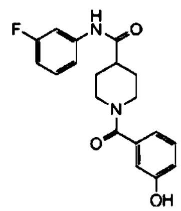

MAGLZ-II-26

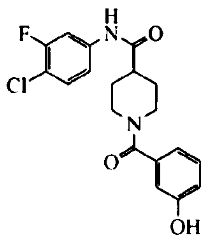

MAGLZ-II-27 et

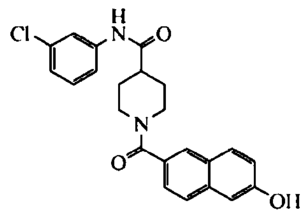

MAGLZ-II-29 .

4. Composé ou sel pharmaceutiquement acceptable de celui-ci selon la revendication 2, où le composé est sélectionné parmi le groupe composé de :

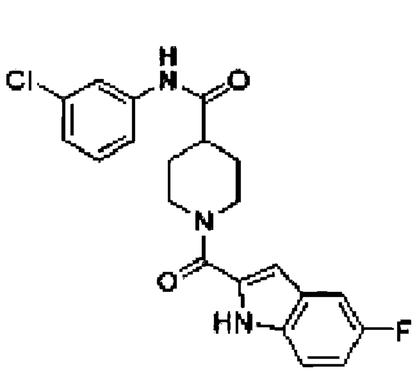

MAGLZ-II-18(a) et

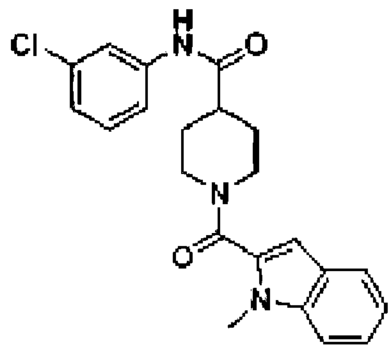

MAGLZ-II-18(c)

5. Composé ou sel pharmaceutiquement acceptable de celui-ci, où le composé est sélectionné parmi le groupe composé de :

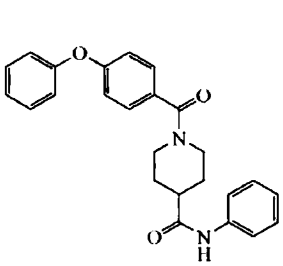

MAGLZ-II-01

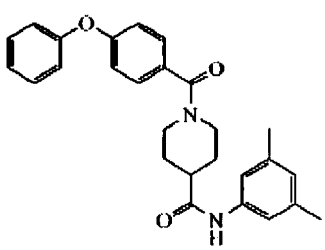

MAGLZ-II-02

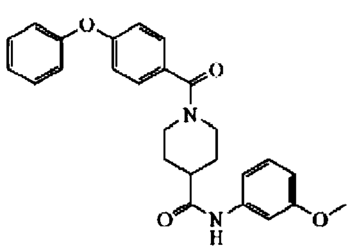

MAGLZ-II-03

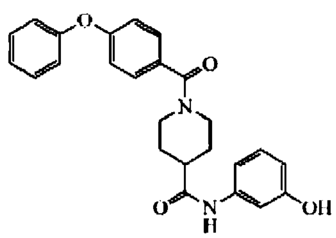

MAGLZ-II-04

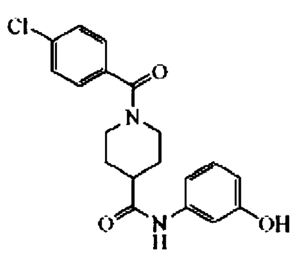

MAGLZ-II-06

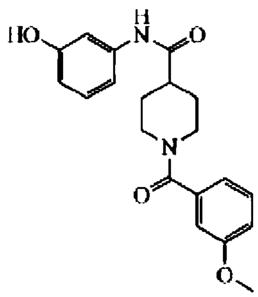

MAGLZ-II-15

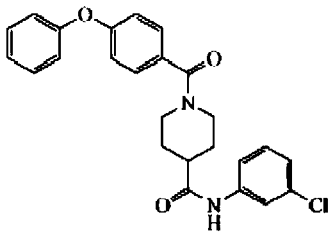

MAGLZ-II-16

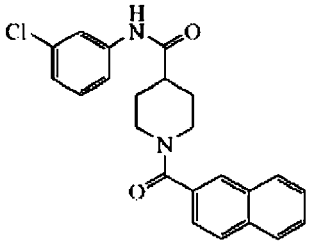

MAGLZ-II-17

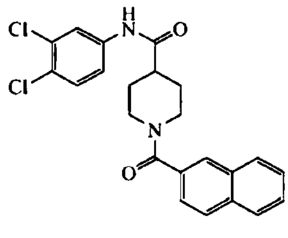

MAGLZ-II-24

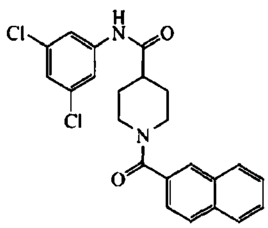

MAGLZ-II-25

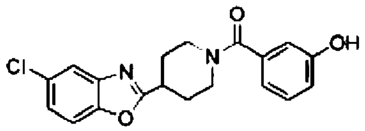

MAGLZ-II-30

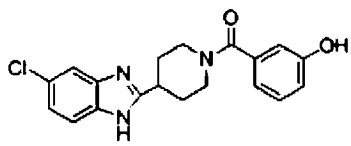

MAGLZ-II-31

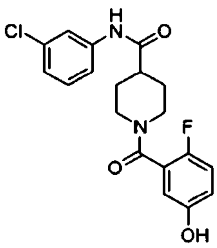

MAGLZ-II-11(a)

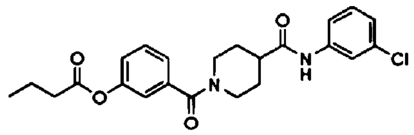

MAGLZ-II-11F

et

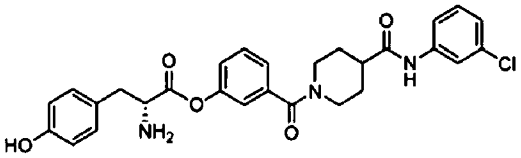

MAGLZ-II-11J

**6.** Procédé de synthèse du composé de la Formule I selon la revendication 1, comprenant :

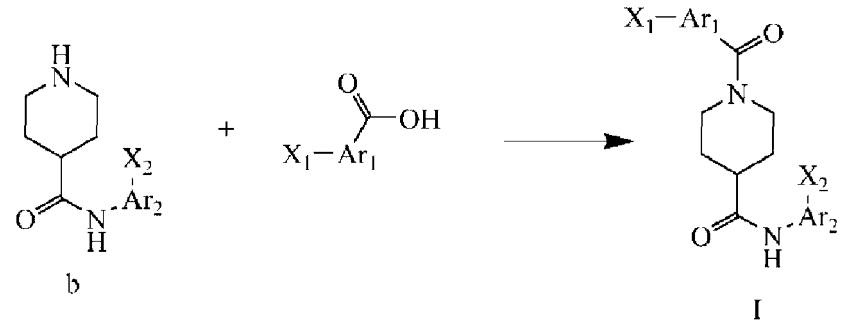

les étapes spécifiques suivantes :

où b ou un sel de b est mis à réagir avec

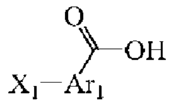

pour obtenir I ; le $X_1$, le $X_2$, le $Ar_1$ et le $Ar_2$ sont décrits dans la revendication 1,
de préférence, où le b ou le sel de b est mis à réagir avec

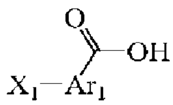

pour obtenir I ; un agent condenseur et un alcali sont ajoutés à cette étape pour faciliter la réaction ; où l'agent condenseur est sélectionné parmi HBTU, DMC, HOBT, HOBT/EDCI, HATU, HATU/DIEPA, DCC, CDI et le chloroformiate d'isopropyle,
de préférence, où le b ou sel de b est obtenu par la déprotection de a ;

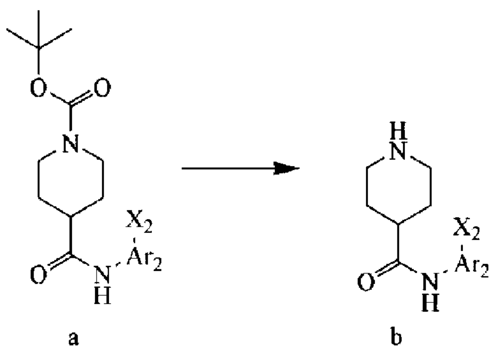

,

de préférence, où le a ou sel de a est obtenu en mettant sm à réagir avec

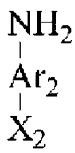

ou un sel de celui-ci ;

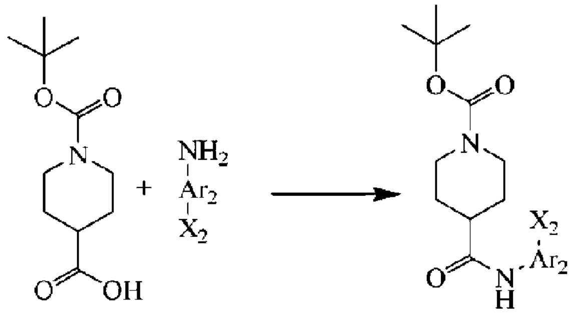

sm a

7. Composition pharmaceutique, comprenant le composé ou sel pharmaceutiquement acceptable de celui-ci selon l'une quelconque des revendications 1-5, et un véhicule pharmaceutiquement acceptable.

8. Composé ou sel pharmaceutiquement acceptable de celui-ci selon l'une quelconque des revendications 1-5, pour une utilisation dans le traitement d'une maladie ou condition médiée par la MAG,
où la maladie ou condition est sélectionnée parmi : des troubles métaboliques, néphrose, émèse ou vomissements et nausées, troubles de l'alimentation, neuropathie, schizophrénie, troubles dépressifs, troubles bipolaires, frémitus, dyskinésie, syndromes de sevrage, lésion cérébrale traumatique, lésion cérébrale non traumatique, lésion de la moelle épinière, crise d'épilepsie, conditions associées à une croissance ou prolifération cellulaire anormale, conditions inflammatoires, conditions du système immunitaire, syndromes de l'intestin irritable, colite ulcéreuse, troubles de stress aigu, anxiété induite par des substances, troubles obsessionnels-compulsifs, troubles d'anxiété, troubles du déficit de l'attention, troubles du déficit de l'attention avec hyperactivité, douleurs, migraine, maladies démyélinisantes, déficits cognitifs.

9. Composé ou sel pharmaceutiquement acceptable de celui-ci pour une utilisation selon la revendication 8, où les conditions associées à une croissance ou prolifération cellulaire anormale sont des tumeurs bénignes ou des cancers.

10. Procédé d'inhibition de la MAGL *in-vitro,* comprenant mettre la MAGL en contact avec le composé ou sel pharmaceutiquement acceptable de celui-ci selon l'une quelconque des revendications 1-5.

11. Composé ou sel pharmaceutiquement acceptable de celui-ci pour une utilisation dans le traitement d'une maladie ou condition médiée par la MAGL, où le composé est sélectionné parmi le groupe composé de :

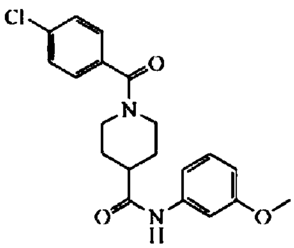

MAGLZ-II-05

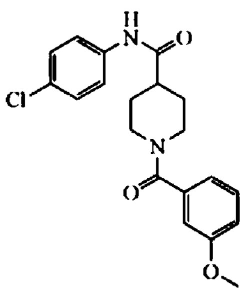

MAGLZ-II-07

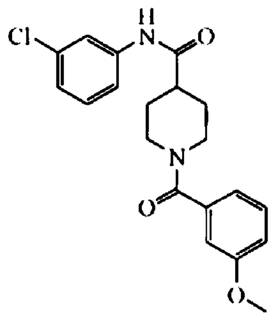

MAGLZ-II-14

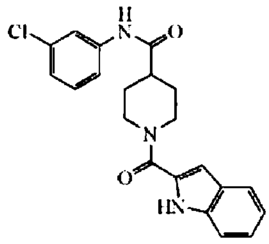

MAGLZ-II-18

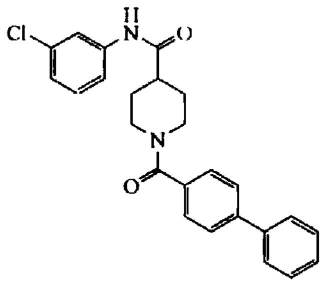

MAGLZ-II-19

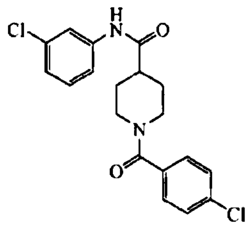

MAGLZ-II-21

,

et

la maladie ou condition médiée par la MAGL est sélectionnée parmi le groupe composé de : troubles métaboliques, néphrose, émèse ou vomissements et nausées, troubles de l'alimentation, neuropathie, schizophrénie, troubles dépressifs, troubles bipolaires, frémitus, dyskinésie, syndromes de sevrage, lésion cérébrale traumatique, lésion cérébrale non traumatique, lésion de la moelle épinière, crise d'épilepsie, conditions associées à une croissance ou prolifération cellulaire anormale, conditions inflammatoires, conditions du système immunitaire, syndromes de l'intestin irritable, colite ulcéreuse, troubles de stress aigu, anxiété induite par des substances, troubles obsessionnels-compulsifs, troubles d'anxiété, troubles de déficit de l'attention, troubles de déficit de l'attention avec hyperactivité, douleurs, migraine, maladies démyélinisantes, déficits cognitifs.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017049295 A1
- US 2017313681 A1
- WO 2018129381 A1
- US 2019202817 A
- WO 2019115660 A1
- WO 2019105915 A1
- WO 2020035424 A
- CN 109593089
- WO 2015149607 A
- CN 105906552
- WO 2013131609 A
- WO 2006069805 A
- WO 2015048570 A


### Non-patent literature cited in the description


- **ZHI ZHUOER et al.** Discovery of Aryl Formyl Piperidine Derivatives as Potent, Reversible, and Selective Monoacylglycerol Lipase Inhibitors. *J. Med. Chem.*, 2020
- **CAREY** ; **SUNDBERG**. ADVANCED ORGANIC CHEMISTRY. Plenum Press, 2000, vol. A
- **CAREY, SUNDBERG**. ADVANCED ORGANIC CHEMISTRY. Plenum Press, 2001, vol. B
- VOGEL'S ENCYCLOPEDIA OF PRACTICAL ORGANIC CHEMISTRY. Longman Scientific and Technical Ltd., 1991, 809-816
- **HELLER**. *Acc. Chem. Res.*, 1990, vol. 23, 128
- **GOODMAN** ; **GILMAN**. The Pharmacological Basis of Therapeutics. Pergamon
- Remington's, Pharmaceutical Sciences. Mack Publishing Co.
- Remingtong's Pharmaceutical Sciences. Mack Publishing Company, 1985, 1418
- *Journal of Pharmaceutical Science*, 1977, vol. 66, 2
- **PORSOLT R D.** Behavioural despair in mice: A primary screening test for antidepressants[J. *Arch. Int. Pharmacolodyn.*, 1977, 229
- **PORSOLT R D** ; **LE PICHON M** ; **JALFRE M.** Depression: a new animal model sensitive to antidepressant treatments[J. *Nature*, 1977, vol. 266 (5604), 730-732